# EUROPEAN PATENT APPLICATION

(11) **EP 4 512 414 A1**
(43) Date of publication of application: **26.02.2025**
(21) Application number: 23792132.5
(22) Date of filing: 18.04.2023
(51) Int. Cl.: A61K 38/26, A61K 47/68, A61K 45/06, A61P 1/00, A61P 29/00, A61P 37/00, C07K 14/605

(54) **COMBINATION THERAPY OF GLP-2 WITH INSULINOTROPIC PEPTIDE, TNF? INHIBITOR OR BOTH FOR PREVENTING OR TREATING INTESTINAL DISEASES**

(30) Priority: 18.04.2022 KR 20220047662
(71) Applicant: Hanmi Pharm. Co., Ltd., Hwaseong-si, Gyeonggi-do 18536 (KR)
(72) Inventor: CHOI, Jae Hyuk, Hwaseong-si, Gyeonggi-do 18469 (KR); LEE, Jin Bong, Hwaseong-si, Gyeonggi-do 18469 (KR); PARK, Eun Jin, Hwaseong-si, Gyeonggi-do 18469 (KR); LEE, Sang Hyun, Hwaseong-si, Gyeonggi-do 18469 (KR); KIM, Sang Yun, Hwaseong-si, Gyeonggi-do 18469 (KR); PARK, Sul Hee, Hwaseong-si, Gyeonggi-do 18469 (KR)
(74) Representative: Mewburn Ellis LLP
(86) International application number: PCT/KR2023/005198
(87) International publication number: WO 2023/204556

(57) **Abstract**

The present disclosure relates to a combination therapy comprising GLP-2, and an insulinotropic peptide, a TNFα inhibitor, or both for preventing, improving, or treating bowel disease.

## Description

### [Technical Field]

The present invention relates to a combination therapy for preventing or treating bowel disease, by administering glucagon-like peptide 2 (GLP-2) in combination with an insulinotropic peptide, a TNFα inhibitor, or both.

### [Background Art]

Bowel disease includes irritable bowel disease, enteritis, inflammatory bowel disease, enterocolitis, colitis, pancreatitis, ileitis, bowel atrophy, bowel damage, *etc.* Among these, inflammatory bowel disease (IBD) is an inflammatory disease characterized by inflammation or ulcers in the gastrointestinal tract, and may exhibit symptoms such as increased expression of inflammatory cytokines, weight loss, shortened colon length, abdominal pain, fever, diarrhea, and/or hematochezia. These symptoms can worsen and improve repeatedly.

Glucagon-like peptide 1 (GLP-1), a type of insulinotropic peptide, is an incretin hormone secreted by L-cells in the ileum and colon. The primary action of GLP-1 is to increase insulin secretion. It causes glucose-dependent secretion of insulin, thereby preventing hypoglycemia. Due to this feature, GLP-1 is applied as a therapy for type 2 diabetes; however, it has a significant limitation for drug development because its half-life in the blood is very short (about 2 minutes). Accordingly, an exemplary GLP-1 agonist developed and marketed is exendin-4, which is a GLP-1 analog purified from the saliva of glia monster lizard. It has higher physiological activity than GLP-1 together with the resistance to dipeptidyl peptidase-4 (DPP-IV), resulting in a longer half-life of 2 to 4 hours compared to GLP-1 (US 5,424,286 A). However, a sufficient duration of physiological activity cannot be expected by increasing the resistance to DPP-IV alone. For example, the exendin-4 (exenatide) currently available in the market still requires administration to patients twice daily via injection, and the side effects such as vomiting and nausea from its administration continue to be a significant burden for patients.

GLP-2 is a peptide hormone consisting of 33 amino acids produced by L-cells in the small intestine in response to ingested nutrients. GLP-2 induces mucosal growth in the small intestine and colon, promotes the growth of intestinal and crypt cells, and inhibits apoptosis. Additionally, GLP-2 increases nutrient absorption in the small intestine and reduces intestinal permeability. It inhibits gastric emptying and gastric acid secretion, increases the intestinal blood flow, and relaxes intestinal smooth muscle.

TNFα is a cytokine that plays a central role in immune responses and is also associated with inflammatory reactions. Abnormal regulation of TNFα is known to occur in various diseases. In order to inhibit such responses caused by TNFα, various TNFα inhibitors have been developed.

### [Disclosure]

### [Technical Problem]

To date, the development of a therapeutic agent for bowel disease using an insulinotropic peptide, GLP-2, and/or a TNFα inhibitor remains insufficient. Therefore, there is a need for the development of a long-acting therapy.

### [Technical Solution]

One object of the present invention is to provide a therapy for preventing, improving, or treating bowel disease by combining GLP-2 with an insulinotropic peptide, a TNFα inhibitor, or both.

Another object of the present invention is to provide a pharmaceutical composition for preventing or treating bowel disease, comprising GLP-2, wherein the pharmaceutical composition is characterized by being administered in combination with an insulinotropic peptide, a TNFα inhibitor, or both.

Still another object of the present invention is to provide a combination which comprises GLP-2, and an insulinotropic peptide, a TNFα inhibitor, or both.

Still another object of the present invention is to provide a pharmaceutical composition for preventing, improving, or treating bowel disease, comprising the combination.

Still another object of the present invention is to provide a pharmaceutical kit for preventing, improving, or treating bowel disease, comprising GLP-2, and an insulinotropic peptide, a TNFα inhibitor, or both.

Still another object of the present invention is to provide a method for preventing, improving, or treating bowel disease, comprising administering and/or using the combination, pharmaceutical composition, or pharmaceutical kit to an individual in need thereof.

Still another object of the present invention is to provide a method for preventing, improving, or treating bowel disease, which comprises administering and/or using a composition containing a pharmaceutically effective amount of GLP-2 in combination with a composition containing a pharmaceutically effective amount of an insulinotropic peptide, a composition containing a pharmaceutically effective amount of a TNFα inhibitor, or both, to an individual in need thereof.

Still another object of the present invention is to provide a use of the combination, pharmaceutical composition, or pharmaceutical kit for preventing, improving, or treating bowel disease; and/or for the preparation of a drug for preventing, improving, or treating bowel disease.

### [Advantageous Effects]

The combination therapy comprising GLP-2, and an insulinotropic peptide, a TNFα inhibitor, or both of the present invention provides an enhanced effect compared to monotherapy and can be effectively used for preventing, improving, or treating bowel disease.

### [Brief Description of the Drawing]

FIG. 1 shows the results of confirming the inhibitory effects of the combined administration of GLP-2 (long-acting GLP-2 derivative conjugate) and an insulinotropic peptide (long-acting GLP-1 derivative conjugate) on M1 polarization (A), macrophage differentiation (B), and monocyte migration (C).
FIG. 2 shows the changes in small intestine length in indomethacin (INN)-induced inflammatory bowel disease rat models following the combined administration of GLP-2 (long-acting GLP-2 derivative conjugate), and an insulinotropic peptide (long-acting GLP-1 derivative conjugate), a TNFα inhibitor (anti-TNFα antibody), or both.
FIG. 3 illustrates the changes in ulcer area in indomethacin-induced inflammatory bowel disease rat models following the combined administration of GLP-2 (long-acting GLP-2 derivative conjugate), and an insulinotropic peptide (long-acting GLP-1 derivative conjugate), a TNFα inhibitor (anti-TNFα antibody), or both.
FIG. 4 shows the changes in colon length in dextran sulfate sodium (DSS)-induced ulcerative colitis mouse models following the combined administration of GLP-2 (long-acting GLP-2 derivative conjugate) and an insulinotropic peptide (long-acting GLP-1 derivative conjugate).
FIG. 5 illustrates the disease activity index (DAI) in DSS-induced ulcerative colitis mouse models following the combined administration of GLP-2 (long-acting GLP-2 derivative conjugate) and an insulinotropic peptide (long-acting GLP-1 derivative conjugate).

### [Detailed Description of Preferred Embodiments]

One aspect of the present invention provides a therapy for preventing, improving, or treating bowel disease by combining GLP-2 with an insulinotropic peptide, a TNFα inhibitor, or both.

One aspect of the present invention provides a composition for preventing, improving, or treating bowel disease, comprising GLP-2, wherein the GLP-2 is characterized by being administered in combination with an insulinotropic peptide, a TNFα inhibitor, or both.

In one embodiment, the present invention provides a pharmaceutical composition for preventing, improving, or treating bowel disease, comprising a pharmaceutically effective amount of GLP-2, wherein the pharmaceutical composition is characterized by being administered in combination with an insulinotropic peptide, a TNFα inhibitor, or both.

In another embodiment, the present invention provides a food composition for preventing or improving bowel disease, comprising GLP-2, wherein the food composition is characterized by being administered in combination with an insulinotropic peptide, a TNFα inhibitor, or both.

In a composition according to any one of the preceding embodiments, the insulinotropic peptide is selected from the group consisting of glucagon-like peptide-1 (GLP-1), exendin-3, exendin-4, an agent, derivative, fragment, and variant thereof, and a combination thereof.

In a composition according to any one of the preceding embodiments, the insulinotropic peptide is an insulinotropic peptide derivative in which the N-terminal histidine residue of the insulinotropic peptide is substituted with imidazoacetyldehistidine, desaminohistidine, β-hydroxyimidazopropionyldehistidine, N-dimethylhistidine, or β-carboxyimidazopropionyldehistidine.

In a composition according to any one of the preceding embodiments, the insulinotropic peptide is native exendin-4; an exendin-4 derivative in which the alpha carbon of the first amino acid of the N-terminus of exendin-4, which is a histidine residue, and the N-terminal amino group attached to the alpha carbon are removed; an exendin-4 derivative in which the N-terminal amino group of exendin-4 is removed; an exendin-4 derivative in which the N-terminal amino group of exendin-4 is substituted with a hydroxy group; an exendin-4 derivative in which the N-terminal amino group of exendin-4 is modified with two methyl groups; an exendin-4 derivative in which the N-terminal amino group of exendin-4 is substituted with a carboxy group; an exendin-4 derivative in which the 12th amino acid (lysine) of exendin-4 is substituted with serine; or an exendin-4 derivative in which the 12th amino (lysine) acid of exendin-4 is substituted with arginine.

In a composition according to any one of the preceding embodiments, the GLP-2 is a native GLP-2 or a GLP-2 derivative.

In a composition according to any one of the preceding embodiments, the GLP-2 derivative is a GLP-2 derivative in which a modification selected from the group consisting of substitution, addition, deletion, chemical modification, and a combination thereof has occurred in at least one amino acid of the sequence of the native GLP-2.

In a composition according to any one of the preceding embodiments, the GLP-2 derivative is a GLP-2 derivative in which a modification has occurred in at least one amino acid of the amino acids at positions 1, 2, 30, and 33 of SEQ ID NO: 1.

In a composition according to any one of the preceding embodiments, the GLP-2 derivative comprises an amino acid sequence represented by General Formula 1 below:
[General Formula 1] X₁X₂DGSFSDEMNTILDNLAARDFINWLIQTX₃₀ITDX₃₄ (SEQ ID NO: 9)
wherein,
X₁ is histidine, imidazoacetyldeshitidine, desaminohistidine, β-hydroxyimidazopropionyldeshitidine, N-dimethylhistidine, or β-carboxyimidazopropionyldeshitidine;
X₂ is alanine, glycine, or 2-aminoisobutyric acid (Aib);
X₃₀ is lysine or arginine; and
X₃₄ is absent, or lysine, arginine, glutamine, histidine, 6-azidolysine, or cysteine;
with the proviso that among the amino acid sequences of General Formula 1, a sequence identical to SEQ ID NO: 1 is excluded.

In a composition according to any one of the preceding embodiments, the GLP-2 derivative comprises an amino acid sequence represented by General Formula 1, in which:
(1) X₁ is imidazoacetyldeshistidine, X₂ is glycine, X₃₀ is lysine, and X₃₄ is cysteine;
(2) X₁ is imidazoacetyldeshistidine, X₂ is glycine, X₃₀ is lysine, and X₃₄ is lysine;
(3) X₁ is imidazoacetyldeshistidine, X₂ is glycine, X₃₀ is arginine, and X₃₄ is lysine;
(4) X₁ is imidazoacetyldeshistidine, X₂ is glycine, X₃₀ is lysine, and X₃₄ is 6-azidolysine;
(5) X₁ is imidazoacetyldeshistidine, X₂ is glycine, X₃₀ is arginine, and X₃₄ is cysteine;
(6) X₁ is imidazoacetyldeshistidine, X₂ is Aib, X₃₀ is lysine, and X₃₄ is cysteine; or
(7) X₁ is histidine, X₂ is Aib, X₃₀ is lysine, and X₃₄ is cysteine.

In a composition according to any one of the preceding embodiments, the GLP-2 derivative comprises an amino acid sequence represented by General Formula 2 below;
[General Formula 2] X₁X₂DGSFSDEMNTILDNLAARDFINWLIQTX₃₀ITDX₃₄ (SEQ ID NO: 10)
wherein,
X₁ is histidine, imidazoacetyldeshitidine, desaminohistidine, β-hydroxyimidazopropionyldeshitidine, N-dimethylhistidine, or β-carboxyimidazopropionyldeshitidine;
X₂ is alanine, glycine, or 2-aminoisobutyric acid (Aib);
X₃₀ is lysine or arginine; and
X₃₄ is any one or more amino acids or any one or more amino acids in which a modification has occurred;
with the proviso that among the amino acid sequences of General Formula 2, the sequence identical to SEQ ID NO: 1 is excluded.

In a composition according to any one of the preceding embodiments, the GLP-2 derivative is a peptide of an amino acid sequence selected from the group consisting of SEQ ID NOS: 2 to 8.

In a composition according to any one of the preceding embodiments, GLP-2 is administered in combination with an insulinotropic peptide.

In a composition according to any one of the preceding embodiments, GLP-2 is administered in combination with a TNFα inhibitor.

In a composition according to any one of the preceding embodiments, GLP-2 is administered in combination with both an insulinotropic peptide and a TNFα inhibitor.

In a composition according to any one of the preceding embodiments, the TNFα inhibitor is a soluble TNF receptor, an anti-TNFα inhibitor or a fragment thereof, or a combination thereof.

In a composition according to any one of the preceding embodiments, the bowel disease is at least one selected from the group consisting of irritable bowel disease, enteritis, inflammatory bowel disease, enterocolitis, colitis, pancreatitis, ileitis, bowel atrophy, and bowel damage.

In a composition according to any one of the preceding embodiments, the inflammatory bowel disease is at least one selected from the group consisting of ulcerative colitis, Crohn's disease, and Behcet's disease.

In a composition according to any one of the preceding embodiments, the composition, when administered to a subject, causes at least one among inhibition of M1 polarization in monocytes, inhibition of macrophage differentiation, and inhibition of monocyte migration.

In a composition according to any one of the preceding embodiments, the composition, when administered to a subject, causes at least one among increased length of the small intestine, decreased inflammation of the small intestine, increased length of the large intestine, and decreased inflammation of the large intestine.

In a composition according to any one of the preceding embodiments, the insulinotropic peptide and GLP-2, the C-terminus is not modified or amidated.

In a composition according to any one of the preceding embodiments, (i) the insulinotropic peptide is in the form of a long-acting conjugate to which a biocompatible material capable of increasing its *in vivo* half-life is bound;
(ii) the GLP-2 is in the form of a long-acting conjugate to which a biocompatible material capable of increasing its *in vivo* half-life is bound; or
(iii) the insulinotropic peptide and GLP-2 are each in the form of a long-acting conjugate to which a biocompatible material capable of increasing their respective in *vivo* half-life is bound.

In a composition according to any one of the preceding embodiments, the conjugate is represented by Formula 1 below:

[Formula 1] X - La - F

wherein,
X is an insulinotropic peptide or GLP-2;
L is a linker containing an ethylene glycol repeating unit;
a is 0 or a natural number wherein when a is 2 or more, each L is independent of the other(s);
F is an immunoglobulin Fc region; and
the "-" represents a covalent bond.

In a composition according to any one of the preceding embodiments, the immunoglobulin Fc region is an aglycosylated IgG4 Fc region.

In a composition according to any one of the preceding embodiments, the F is a dimer consisting of two polypeptide chains, and one end of L is linked to only one of the two polypeptide chains.

In a composition according to any one of the preceding embodiments, the L is polyethylene glycol.

In a composition according to any one of the preceding embodiments, the formula weight of the ethylene glycol repeating unit moiety in L is in the range of 1 kDa to 100 kDa.

In a composition according to any one of the preceding embodiments, the composition further comprises a pharmaceutically acceptable carrier, an excipient, or a diluent.

In a composition according to any one of the preceding embodiments, (i) GLP-2 and an insulinotropic peptide; (ii) GLP-2 and a TNFα inhibitor; or (iii) GLP-2, an insulinotropic peptide, and a TNFα inhibitor are administered in combination simultaneously, sequentially, or in reverse order.

Still another aspect of the present invention provides a combination comprising GLP-2, and an insulinotropic peptide, a TNFα inhibitor, or both.

Still another aspect of the present invention provides a pharmaceutical composition for preventing, improving, or treating bowel disease, comprising the combination.

Still another aspect of the present invention provides a pharmaceutical kit for preventing, improving, or treating bowel disease, comprising GLP-2, and an insulinotropic peptide, a TNFα inhibitor, or both.

Still another aspect of the present invention provides a method for preventing, improving, or treating bowel disease, comprising administering and/or using the combination, pharmaceutical composition, or pharmaceutical kit to an individual in need thereof.

Still another aspect of the present invention is a method for preventing, improving, or treating bowel disease, comprising administering and/or using a composition containing a pharmaceutically effective amount of GLP-2 in combination with a composition containing a pharmaceutically effective amount of an insulinotropic peptide, a composition containing a pharmaceutically effective amount of a TNFα inhibitor, or both, to an individual in need thereof.

Still another aspect of the present invention provides a use of the combination, pharmaceutical composition, or pharmaceutical kit for preventing, improving, or treating bowel disease; and/or for the preparation of a drug for preventing, improving, or treating bowel disease.

### [Mode for Carrying Out the Invention]

Hereinafter, the present invention will be described in more detail.

Meanwhile, the description and embodiment disclosed herein may also be applied to other descriptions and other embodiments, respectively. In other words, all combinations of the various elements disclosed herein fall within the scope of the present invention. In addition, it cannot be said that the scope of the present invention is limited by the specific description described below.

In addition, those skilled in the art will recognize, or be able to ascertain using no more than routine experimentation, many equivalents to the specific aspects of the present invention described in this application. In addition, such equivalents are intended to be included in the present invention.

Throughout this specification, conventional one-letter and three-letter codes for naturally-occurring amino acids are used as well as generally accepted three letter codes for other amino acids such as 2-aminoisobutyric acid (Aib) and 6-azidolysine (AZK) are used. In addition, amino acids referred to by abbreviations herein are described according to the IUPAC-IUB nomenclature.

| | |
|---|---|
| Alanine (Ala, A) | Arginine (Arg, R) |
| Asparagine (Asn, N) | Aspartic acid (Asp, D) |
| Cysteine (Cys, C) | Glutamic acid (Glu, E) |
| Glutamine (Gln, Q) | Glycine (Gly, G) |
| Histidine (His, H) | Isoleucine (Ile, I) |
| Leucine (Leu, L) | Lysine (Lys, K) |
| Methionine (Met, M) | Phenylalanine (Phe, F) |
| Proline (Pro, P) | Serine (Ser, S) |
| Threonine (Thr, T) | Tryptophan (Trp, W) |
| Tyrosine (Tyr, Y) | Valine (Val, V) |

An aspect embodying the present invention provides a pharmaceutical composition for preventing or treating bowel disease, comprising GLP-2, wherein the pharmaceutical composition is characterized by being administered in combination with an insulinotropic peptide, a TNFα inhibitor, or both.

In one embodiment of the present invention, the pharmaceutical composition may be a pharmaceutical composition for preventing or treating bowel disease, comprising a pharmaceutically effective amount of GLP-2, wherein the pharmaceutical composition is characterized by being administered in combination with an insulinotropic peptide (such as a pharmaceutically effective amount of an insulinotropic peptide), a TNFα inhibitor (such as a pharmaceutically effective amount of a TNFα inhibitor), or both, but is not limited thereto.

Another aspect of the present invention provides a use of GLP-2 in combination with an insulinotropic peptide, TNFα inhibitor, or both in the prevention, improvement, or treatment of bowel disease.

Another embodiment provides a pharmaceutical composition for preventing, improving, or treating bowel disease, comprising GLP-2, and an insulinotropic peptide, TNFα inhibitor, or both.

Specifically, one aspect of the present invention provides a combination, pharmaceutical composition, or pharmaceutical kit comprising GLP-2, and an insulinotropic peptide, TNFα inhibitor, or both. One embodiment provides a combination, pharmaceutical composition, or pharmaceutical kit for preventing, improving, or treating bowel disease.

As used herein, the term "combination" refers to the use of GLP-2, and an insulinotropic peptide, a TNFα inhibitor, or both for combined administration, and may be understood as the same meaning as "combined use". This also includes the forms of a pharmaceutical composition and a pharmaceutical kit in which GLP-2 is used in combination with an insulinotropic peptide, a TNFα inhibitor, or both, but is not limited thereto.

Specifically, (i) GLP-2, and an insulinotropic peptide; (ii) GLP-2 and a TNFα inhibitor; or (iii) GLP-2, an insulinotropic peptide, and a TNFα inhibitor may be administered in combination simultaneously, sequentially, or in reverse order.

The combination may be:
i) one in which GLP-2, and an insulinotropic peptide, a TNFα inhibitor, or both are administered as a mixture or in separate preparations;
ii) one in which a pharmaceutical composition comprising GLP-2 is administered in combination with a pharmaceutical composition comprising an insulinotropic peptide, a pharmaceutical composition comprising a TNFα inhibitor, or both simultaneously, sequentially, or in reverse order; or
iii) one in which a pharmaceutical composition comprising two substances selected from GLP-2, an insulinotropic peptide, and a TNFα inhibitor in the form of a mixture or separate preparations is administered in combination with the remaining substance simultaneously, sequentially, or in reverse order, but is not limited thereto.

The combination or composition may be:
a) (i) administering GLP-2 and an insulinotropic peptide as a mixture, (ii) administering GLP-2 and a TNFα inhibitor as a mixture, or (iii) administering GLP-2, an insulinotropic peptide, and a TNFα inhibitor as a mixture;
b) administering GLP-2 and an insulinotropic peptide in separate preparations, (ii) administering GLP-2 and a TNFα inhibitor in separate preparations, or (iii) administering GLP-2, an insulinotropic peptide, and a TNFα inhibitor in separate preparations; or
c) administering GLP-2 and an insulinotropic peptide as a mixture and a TNFα inhibitor in a separate form, (ii) administering GLP-2 and a TNFα inhibitor as a mixture and an insulinotropic peptide in a separate form, or (iii) administering an insulinotropic peptide and a TNFα inhibitor as a mixture and GLP-2 in a separate form, but is not limited thereto.

In the case where they are in separate preparations as in b) above, (i) GLP-2 and an insulinotropic peptide; (ii) GLP-2 and a TNFα inhibitor; or (iii) GLP-2, an insulinotropic peptide, and a TNFα inhibitor may each be formulated together in separate preparations or formulated as separate preparations, and these formulations may be administered simultaneously, individually, sequentially, or in reverse order.

In addition, in the case where they are in separate preparations as in c) above, the mixture, and an insulinotropic peptide, GLP-2, or a TNFα inhibitor may each be formulated together in separate preparations or formulated as separate preparations, and these formulations may be administered simultaneously, individually, sequentially, or in reverse order.

As used herein, the term "combined administration", "administered in combination", or "administering in combination" means not only simultaneous administration, but is also to be understood as an administration form in which GLP-2, and an insulinotropic peptide, a TNFα inhibitor, or both act together on a subject so that each substance (the insulinotropic peptide, GLP-2, and/or the TNFα inhibitor) can perform a function at a level equal to or higher than its original function. Accordingly, when the term "use in combination" is used herein, this is to be understood that the use in combination refers to simultaneous, individual, sequential, or reverse order administration, and the order is unlimited. When the administration is sequential, reverse order, or individual, the order of administration is not particularly limited, but the administration interval of the second or subsequent ingredients is to be such that the beneficial effects of the combination is not lost.

As used herein, the term "composition comprising a combination" may be the combination containing GLP-2, and an insulinotropic peptide, a TNFα inhibitor, or both, or may comprise the combination and have a therapeutic use, but is not limited thereto. For example, the composition may have a use for preventing, improving, or treating bowel disease, but is not limited thereto. As used herein, the term "composition comprising a combination" may be used interchangeably with "composition".

The composition comprising a combination according to the present invention is for administration of GLP-2 in combination with an insulinotropic peptide, a TNFα inhibitor, or both, and GLP-2, and the insulinotropic peptide, the TNFα inhibitor, or both may be prepared as a single preparation or as two or more separate preparations. Specifically, the composition may be one in which GLP-2 is administered with an insulinotropic peptide, a TNFα inhibitor, or both simultaneously, individually, sequentially, or in reverse order, but the administration is not limited thereto.

As used herein, the term "kit" may comprise a combination or composition according to the present invention to administer GLP-2 in combination with an insulinotropic peptide, a TNFα inhibitor, or both. Specifically, the kit according to the present invention may comprise a) (i) GLP-2 and an insulinotropic peptide, (ii) GLP-2 and a TNFα inhibitor, or (iii) GLP-2, an insulinotropic peptide, and a TNFα inhibitor, formulated as a single preparation; b) (i) separate preparations of GLP-2 and an insulinotropic peptide, (ii) separate preparations of GLP-2 and a TNFα inhibitor, or (iii) separate preparations of GLP-2, an insulinotropic peptide, and a TNFα inhibitor; or c) (i) a single preparation of GLP-2 and an insulinotropic peptide and a separate preparation of a TNFα inhibitor, (ii) a single preparation of GLP-2 and a TNFα inhibitor and a separate preparation of an insulinotropic peptide, or (iii) a single preparation of an insulinotropic peptide and a TNFα inhibitor and a separate preparation of GLP-2, and may further comprise substances required for the combined administration of the two or more substances, but the components are not limited thereto.

The present invention has confirmed that the effect of preventing, improving, or treating bowel disease is dramatically enhanced when using GLP in combination with an insulinotropic peptide, a TNFα inhibitor, or both compared to that when using an insulinotropic peptide, GLP-2, and a TNFα inhibitor alone, and thus provides the method of using the combination therapy.

The "insulinotropic peptide" refers to a peptide having an insulin-secreting function, and can stimulate the synthesis or expression of insulin in pancreatic beta cells. The insulinotropic peptide may be, for example, glucagon-like peptide-1 (GLP-1), exendin-3, or exendin-4, but is not limited thereto. The insulinotropic peptide includes not only a native insulinotropic peptide, but also precursors, agonists, derivatives, fragments, and variants thereof, and also include long-acting conjugates to which biocompatible substances capable of increasing their *in vivo* half-life are bound. The insulinotropic peptide may be contained in the pharmaceutical composition in a pharmaceutically effective amount.

GLP-1 is a hormone secreted from the small intestine and generally promotes insulin biosynthesis and secretion, inhibits glucagon secretion, and promotes intracellular glucose uptake. In the small intestine, glucagon precursors are broken down into three peptides: glucagon, GLP-1, and GLP-2. Here, GLP-1 means GLP-1 (1-37), which is a form without having an insulin-secreting function, and is processed into a GLP-1 (7-37) form to become active GLP-1 (7-37). The amino acid sequence of GLP-1 (7-37) is as follows.

### GLP-1 (7-37):

HAEGT FTSDV SSYLE GQAAK EFIAW LVKGR G (SEQ ID NO: 32)

Exendin-3 and exendin-4 are GLP-1 analogs or derivatives that consist of 39 amino acids having 53% amino acid sequence similarity with GLP-1, and correspond to insulinotropic peptides. The amino acid sequences of exendin-3 and exendin-4 are as follows.

### Exendin-3:

HSDGT FTSDL SKQME EEAVR LFIEW LKNGG PSSGA PPPS (SEQ ID NO: 33)

### Exendin-4:

HGEGT FTSDL SKQME EEAVR LFIEW LKNGG PSSGA PPPS (SEQ ID NO: 34)

The insulinotropic peptide derivative may have an insulin-secreting function and have at least 80%, 85%, 90%, 95%, 96%, 97%, 98%, or 99% or more homology or identity to the native insulinotropic peptide in amino acid sequence, and/or may be in a form in which some groups of an amino acid residue in the insulinotropic peptide may be chemically substituted (for example, alpha-methylation and alpha-hydroxylation), removed (for example, deamination) or modified (for example, N-methylation), but is limited thereto.

In a specific embodiment, the insulinotropic peptide derivative of the present invention may be prepared by a method in which the alpha amino group of N-terminal histidine is removed, a method in which the N-terminal amino group is substituted with a hydroxyl group or a carboxyl group and synthesis is performed, a method in which the alpha-carbon of N-terminal histidine and the N-terminal amino group bound to the alpha-carbon are removed to leave only the imidazo-acetyl functional group, a method in which the N-terminal amino group is modified with two methyl groups, and the like. The insulinotropic peptide derivative prepared through the methods may be an insulinotropic peptide derivative in which the N-terminal histidine residue of the insulinotropic peptide is substituted with imidazoacetyldeshistidine, desaminohistidine, β-hydroxyimidazopropionyldeshistidine, N-dimethylhistidine, or β-carboxyimidazopropionyldeshistidine.

In an embodiment, the insulinotropic peptide derivative may be a GLP-1 derivative, and the GLP-1 derivative includes exendin-3, exendin-4, or derivatives thereof, but is not limited thereto.

In addition, an example of the insulinotropic peptide derivative may be a derivative obtained by chemically modifying the N-terminal amino group or amino acid residue of exendin-4, and may be imidazoacetyl-deshistidyl-exendin-4 (CA-exendin-4) in which the alpha-carbon of the histidine residue, which is the first N-terminal amino acid of exendin-4, and the N-terminal amino group bound to the alpha-carbon are removed; desaminohistidyl exendin-4 (DA-exendin-4) in which the N-terminal amino group of exendin-4 is removed; β-hydroxyimidazopropionyldeshistidyl exendin-4 (HY-exendin-4) in which the N-terminal amino group of exendin-4 is substituted with a hydroxyl group; N-dimethylhistidyl exendin-4 (DM-exendin-4) in which the N-terminal amino group of exendin-4 is modified with two methyl groups; or β-carboxyimidazopropionyl-deshistidyl exendin-4 (CX-exendin-4) in which the N-terminal amino group of exendin-4 is substituted with a carboxyl group; an exendin-4 derivative in which the twelfth amino acid (lysine) of exendin-4 is substituted with serine; or an exendin-4 derivative in which the twelfth amino acid (lysine) of exendin-4 is substituted with arginine, but is not limited thereto.

The insulinotropic peptide fragment refers to a form in which one or more amino acids are removed from and/or added to the N-terminus or C-terminus of the native insulinotropic peptide, and the added amino acid may also be an amino acid that does not exist in nature (for example, a D-type amino acid).

The insulinotropic peptide variant refers to a peptide that differs from the native insulinotropic peptide by one or more amino acids, exhibits at least 80%, 85%, 90%, 95%, 96%, 97%, 98%, or 99% or more homology or identity to the native insulinotropic peptide, and has an insulin-secreting function.

The insulinotropic peptide agonist refers to a substance that exhibits the same biological activity as the insulinotropic peptide by binding to an *in vivo* receptor of the insulinotropic peptide regardless of the structure of the insulinotropic peptide.

The preparation methods used for the derivative, fragment, variant, and agonist of the present invention, respectively, may be used independently or in combination. For example, insulinotropic peptides in which one or more amino acids are different and the N-terminal amino acid residue is deaminated are also included in the present invention.

As used herein, the term "glucagon-like peptide-2" or "GLP-2" is an agonist of the glucagon-like peptide-2 receptor, and may be in the form of a polypeptide or in the form of a long-acting conjugate to which a biocompatible substance capable of increasing the *in vivo* half-life of the polypeptide is bound, but is not limited thereto.

In the present invention, glucagon-like peptide-2 or GLP-2 is a concept that includes not only sequences identical to native human GLP-2 but also GLP-2 derivatives as well as long-acting conjugates in which biocompatible substances are bound to native GLP-2 or GLP-2 derivatives. The GLP-2 may be contained in the composition or in a separate composition in a pharmaceutically effective amount so as to be administered or used in combination with a pharmaceutical composition comprising an insulinotropic peptide.

The amino acid sequence of native GLP-2 is as follows.

### GLP-2 (1-33):

HADGSFSDEMNTILDNLAARDFINWLIQTKITD (SEQ ID NO: 1)

As used herein, the term "agonist of GLP-2 receptor" refers to a substance that binds to an *in vivo* or isolated human glucagon-like peptide-2 receptor to exhibit physiological activity equivalent or similar to that of native GLP-2. For example, the GLP-2 agonist may include native GLP-2 or GLP-2 derivatives.

As used herein, the term "GLP-2 derivative" includes a peptide having one or more differences in amino acid sequence compared to native GLP-2; a peptide modified through modification of the native GLP-2 sequence; and/or a mimic of native GLP-2 that, like native GLP-2, has a function for preventing, treating, and/or improving bowel disease. Specifically, the GLP-2 derivative may be one in which at least one or more amino acids in the native GLP-2 sequence subjected to modification selected from the group consisting of substitution, addition, deletion, modification, and a combination thereof, but is not limited thereto.

The added amino acid can also be a unnatural amino acid (for example, a D-type amino acid), and substitution with a unnatural amino acid in addition to a natural amino acid is also possible. The added amino acid sequence may be derived from native GLP-2, but is not limited thereto. In addition, in the present invention, modification of amino acids may mean that some groups of amino acid residues are chemically substituted (for example, alpha-methylation, alpha-hydroxylation, and substitution with an azido group), removed (for example, deamination), and/or modified (for example, N-methylation) together with; or independently of the substitution, addition, or removal of at least one amino acid or combinations thereof, but is not limited thereto.

In a specific embodiment, the GLP-2 derivative of the present invention may have at least 80%, 85%, 90%, 95%, 96%, 97%, 98%, or 99% or more homology or identity to native GLP-2 in amino acid sequence, and/or may be in a form in which some groups of an amino acid residue in GLP-2 are chemically substituted (for example, alpha-methylation, alpha-hydroxylation, and substitution with an azido group), removed (for example, deamination), and/or modified (for example, N-methylation), but is not limited thereto.

In a specific embodiment, the N-terminal amino group of GLP-2 of the present invention may be substituted, removed, or modified, but is not limited thereto. In order to prevent binding to the N-terminus, which is an important site for the *in vivo* activity of GLP-2, during preparation of a long-acting conjugate, GLP-2 of the present invention may be prepared by a method in which the alpha amino group of the N-terminal histidine is removed, a method in which the N-terminal amino group is substituted with a hydroxyl group or a carboxyl group and synthesis is performed, a method in which the alpha-carbon of the N-terminal histidine and the N-terminal amino group bound to the alpha-carbon are removed to leave only the imidazoacetyl functional group, a method in which the N-terminal amino group is modified with two methyl groups, and the like.

Specifically, the GLP-2 derivative may be imidazoacetyl-deshistidyl-GLP-2 (CA-GLP-2) in which the alpha-carbon of the histidine residue, which is the first amino acid at the N-terminus of GLP-2, and the N-terminal amino group bound to the alpha-carbon are removed; desaminohistidyl GLP-2 (DA-GLP-2) in which the N-terminal amino group of GLP-2 is removed; β-hydroxyimidazopropionyldeshistidyl GLP-2 (HY-GLP-2) in which the N-terminal amino group of GLP-2 is substituted with a hydroxyl group; N-dimethylhistidyl GLP-2 (DM-GLP-2) in which the N-terminal amino group of GLP-2 is modified with two methyl groups; or β-carboxyimidazopropionyl-deshistidyl GLP-2 (CX-GLP-2) in which the N-terminal amino group of GLP-2 is substituted with a carboxyl group, but is not limited thereto. As a non-limiting example, the structures of the substances used to prepare GLP-2 derivatives are as follows.

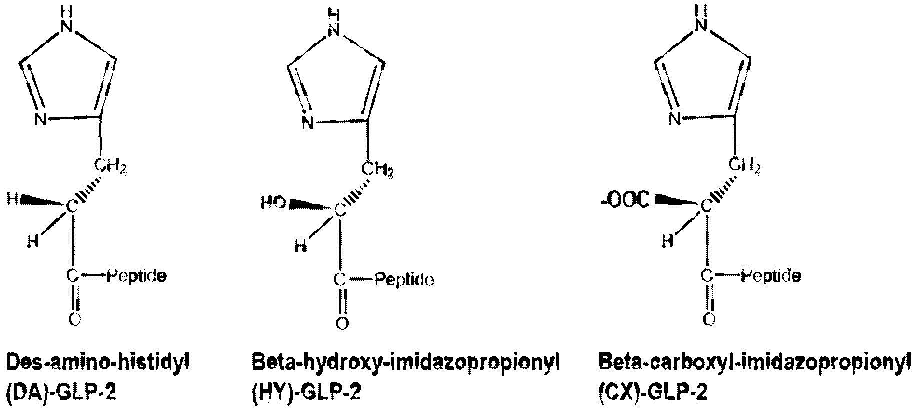

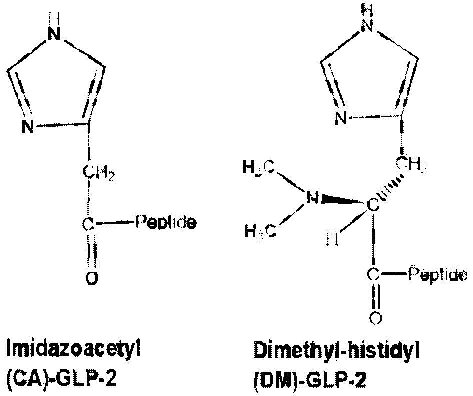

Herein, imidazoacetyldeshistidyl (dine), desaminohistidyl (dine), β-hydroxyimidazopropionyldeshistidyl (dine), N-dimethylhistidyl (dine), and β-carboxyimidazopropionyldeshistidyl (dine) may be used in the same meaning as imidazoacetyl, des-amino-histidyl, β-hydroxy-imidazopropionyl, dimethyl-histidyl, and β-carboxyl-imidazopropionyl, respectively.

In a specific embodiment, the GLP-2 derivative may be subjected to modification of at least one amino acid among amino acids at positions 1, 2, 30, and 33 in SEQ ID NO: 1, but is not limited thereto. Specifically, the modification may be modification selected from the group consisting of substitution, addition, removal, and modification of at least one amino acid, and combinations thereof, the amino acid added at this time can also be a unnatural amino acid (for example, a D-type amino acid), and substitution with a unnatural amino acid in addition to a natural amino acid is also possible. The added amino acid sequence may be derived from native GLP-2, but is not limited thereto. In addition, in the present invention, modification of amino acids may mean that some groups of amino acid residues are chemically substituted (for example, alpha-methylation, alpha-hydroxylation, substitution with an azido group), removed (for example, deamination), and/or modified (for example, N-methylation) together with; or independently of the substitution, addition, or removal of at least one amino acid or combinations thereof, but is not limited thereto.

In a specific embodiment, the GLP-2 derivative may include an amino acid sequence represented by the following General Formula 1, but is not limited thereto:
[General Formula 1] X₁X₂DGSFSDEMNTILDNLAARDFINWLIQTX₃₀ITDX₃₄ (SEQ ID NO: 9)
wherein,
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or 2-aminoisobutyric acid (Aib);
X₃₀ is lysine or arginine; and
X₃₄ is absent or is lysine, arginine, glutamine, histidine, 6-azidolysine, or cysteine.

In another specific embodiment, the GLP-2 derivative may include an amino acid sequence represented by the following General Formula 2, but is not limited thereto:
[General Formula 2] X₁X₂DGSFSDEMNTILDNLAARDFINWLIQTX₃₀ITDX₃₄ (SEQ ID NO: 10)
wherein,
X₁ is histidine, imidazoacetyldeshistidine, desaminohistidine, β-hydroxyimidazopropionyldeshistidine, N-dimethylhistidine, or β-carboxyimidazopropionyldeshistidine;
X₂ is alanine, glycine, or 2-aminoisobutyric acid (Aib);
X₃₀ is lysine or arginine; and
X₃₄ is one or more arbitrary amino acids or one or more arbitrary amino acids subjected to variation.

Specifically, the amino acid may be a natural amino acid or a unnatural amino acid, and the modification of amino acid is as described above.

In addition, among the amino acid sequences represented by General Formula 1 or 2, the sequence identical to SEQ ID NO: 1 may be excluded from the GLP-2 derivatives, but the GLP-2 derivative is not limited thereto.

Specifically, the GLP-2 derivative of the present invention may have a substitution of alanine, the second amino acid of native GLP-2, with glycine or Aib (2-aminoisobutyric acid); substitution of lysine, the 30^{th} amino acid, with arginine; or a combination thereof, but is not limited thereto. In addition, the GLP-2 derivative may have a thiol group (for example, cysteine), an amino group (for example, lysine, arginine, glutamine, or histidine), or an azide group (for example, 6-azidolysine) introduced at the C-terminus (for example, the 33^{rd} amino acid), but the GLP-2 derivative is not limited thereto.

When a long-acting conjugate of a GLP-2 derivative is prepared, bonding occurs at the introduced group, allowing a GLP-2 conjugate having a selectively controlled binding position to be prepared. Specifically, one end of the linker may be bound to the hydroxyl group, thiol group, amino group, or azido group of the GLP-2 derivative, and to the other end of the linker, a substance capable of increasing the *in vivo* half-life (for example, an immunoglobulin Fc region) may be bound. The thiol group, amino group, or azido group may be introduced by adding an amino acid to GLP-2, but the introduction is not limited thereto. The thiol group may be introduced by adding cysteine (C) to GLP-2; the amino group may be introduced by adding lysine (K), arginine (R), glutamine (Q), or histidine (H) to GLP-2; and the azido group may be introduced by adding 6-azidolysine (AZK) to GLP-2, but the introduction is not limited thereto.

Specifically, the GLP-2 derivative according to the present invention may have at least one residue that is cysteine, lysine, arginine, glutamine, histidine, or 6-azidolysine, but is not limited thereto.

Specifically, the GLP-2 derivative of the present invention includes substitution of alanine, the second amino acid of native GLP-2, with glycine and introduction of a thiol group (for example, cysteine) into the C-terminus, more specifically, may include imidazoacetyldeshistidine in which the alpha-carbon of the histidine residue, the first N-terminal amino acid, and the N-terminal amino group bound to the alpha-carbon are removed, and may have, for example, the amino acid sequence of SEQ ID NO: 2, but is not limited thereto.

Specifically, the GLP-2 derivative of the present invention includes substitution of alanine, the second amino acid of native GLP-2, with glycine and introduction of an amino group (for example, lysine) into the C-terminus, more specifically, may include imidazoacetyldeshistidine in which the alpha-carbon of the histidine residue, the first N-terminal amino acid, and the N-terminal amino group bound to the alpha-carbon are removed, and may have, for example, the amino acid sequence of SEQ ID NO: 3, but is not limited thereto.

Specifically, the GLP-2 derivative of the present invention includes substitution of alanine, the second amino acid of native GLP-2, with glycine, substitution of lysine, the 30^{th} amino acid of native GLP-2, with arginine, and introduction of an amino group (for example, lysine) into the C-terminus, more specifically, may include imidazoacetyldeshistidine in which the alpha-carbon of the histidine residue, the first N-terminal amino acid, and the N-terminal amino group bound to the alpha-carbon are removed, and may have, for example, the amino acid sequence of SEQ ID NO: 4, but is not limited thereto.

Specifically, the GLP-2 derivative of the present invention includes substitution of alanine, the second amino acid of native GLP-2, with glycine and introduction of an azide group (for example, 6-azidolysine) into the C-terminus, more specifically, may include imidazoacetyldeshistidine in which the alpha-carbon of the histidine residue, the first N-terminal amino acid, and the N-terminal amino group bound to the alpha-carbon are removed, and may have, for example, the amino acid sequence of SEQ ID NO: 5, but is not limited thereto.

Specifically, the GLP-2 derivative of the present invention includes substitution of alanine, the second amino acid of native GLP-2, with glycine, substitution of lysine, the 30^{th} amino acid of native GLP-2, with arginine, and introduction of a thiol group (for example, cysteine) into the C-terminus, more specifically may include imidazoacetyldeshistidine in which the alpha-carbon of the histidine residue, the first N-terminal amino acid, and the N-terminal amino group bound to the alpha-carbon are removed, and may have, for example, the amino acid sequence of SEQ ID NO: 6, but is not limited thereto.

Specifically, the GLP-2 derivative of the present invention includes substitution of alanine, the second amino acid of native GLP-2, with 2-aminoisobutyric acid and introduction of a thiol group (for example, cysteine) into the C-terminus, and may have, for example, the amino acid sequence of SEQ ID NO: 8, and more specifically, the GLP-2 derivative may include imidazoacetyldeshistidine in which the alpha-carbon of the histidine residue, the first N-terminal amino acid, and the N-terminal amino group bound to the alpha-carbon are removed, and may have, for example, the amino acid sequence of SEQ ID NO: 7, but is not limited thereto.

The GLP-2 derivatives of SEQ ID NOs: 2 to 8 are shown in Table 1 below.

**[Table 1]**

| Name | Sequence | SEQ ID NO: |
|---|---|---|
| CA GLP-2 KC | *_{ca}*HGDGSFSDEMNTILDNLAARDFINWLIQTKITDC | 2 |
| CA GLP-2 KK | *_{ca}*HGDGSFSDEMNTILDNLAARDFINWLIQTKITDK | 3 |
| CA GLP-2 RK | *_{ca}*HGDGSFSDEMNTILDNLAARDFINWLIQTRITDK | 4 |
| CA GLP-2 K_{AZ}K | *_{ca}*HGDGSFSDEMNTILDNLAARDFINWLIQTKITD_{AZ}K | 5 |
| CA GLP-2 RC | *_{ca}*HGDGSFSDEMNTILDNLAARDFINWLIQTRITDC | 6 |
| CA GLP-2 Aib | *_{ca}*HAibDGSFSDEMNTILDNLAARDFINWLIQTKITDC | 7 |
| GLP-2 Aib | HAibDGSFSDEMNTILDNLAARDFINWLIQTKITDC | 8 |

In Table 1, _{ca}H indicates that histidine is substituted with imidazoacetyldeshistidine, Aib indicates 2-aminoisobutyric acid, and AZK indicates 6-azido-L-lysine.

GLP-2 according to the present invention may be a peptide including the specific sequence described above or a peptide consisting of (or essentially consisting of) the specific sequence described above, but is not limited thereto.

Meanwhile, even if a peptide or GLP-2 "consisting of (composed of) a specific sequence number" is described herein, it does not exclude addition of meaningless sequences before and after the amino acid sequence of the corresponding sequence number, or naturally occurring mutations, or silent mutations thereof as long as the peptide or GLP-2 exhibits activity the same as or equivalent to that of a peptide or GLP-2 composed of the amino acid sequence of the corresponding sequence number, and it is obvious that a peptide or GLP-2 having such sequence additions or mutations also falls within the scope of the present application.

Specifically, the GLP-2 derivative may be a compound of General Formula 1 or General Formula 2, in which (1) X₂ may be glycine or Aib; (2) X₃₀ may be lysine or arginine, or (3) X₂ may be glycine or Aib and X₃₀ may be lysine or arginine, but the GLP-2 derivative is not limited thereto.

Specifically, the GLP-2 derivative may be,
(1) X₁ may be imidazoacetyldeshistidine, X₂ may be glycine, X₃₀ may be lysine, and X₃₄ may be cysteine;
(2) X₁ may be imidazoacetyldeshistidine, X₂ may be glycine, X₃₀ may be lysine, and X₃₄ may be lysine;
(3) X₁ may be imidazoacetyldeshistidine, X₂ may be glycine, X₃₀ may be arginine, and X₃₄ may be lysine;
(4) X₁ may be imidazoacetyldeshistidine, X₂ may be glycine, X₃₀ may be lysine, and X₃₄ may be 6-azidolysine;
(5) X₁ may be imidazoacetyldeshistidine, X₂ may be glycine, X₃₀ may be arginine, and X₃₄ may be cysteine;
(6) X₁ may be imidazoacetyldeshistidine, X₂ may be Aib, X₃₀ may be lysine, and X₃₄ may be cysteine; or
(7) X₁ may be histidine, X₂ may be Aib, X₃₀ may be lysine, and X₃₄ may be cysteine, but the GLP-2 derivative is not limited thereto.

These modifications for the preparation of agonists, fragments, variants, and derivatives of native GLP-2 in the present invention include all modifications using L-type or D-type amino acids and/or unnatural amino acids; and/or modifications by modification or post-translational modification (for example, methylation, acylation, ubiquitination, and intramolecular covalent bond) of the native sequence.

In the present invention, the GLP-2 or GLP-2 derivative may be chemically modified at the N-terminus and/or C-terminus, protected with organic groups, or have additional amino acids added to, for example, the termini, in order to protect it from proteolytic enzymes *in vivo* and to increase its stability.

In particular, in the case of chemically synthesized peptides, since the N- and C-termini are charged, the N-terminus may be acetylated and/or the C-terminus may be amidated in order to remove these charges, but the modification is not particularly limited thereto. Specifically, in the present invention, GLP-2 or a GLP-2 derivative may have its C-terminus unmodified or amidated, but is not limited thereto.

As used herein, the term "tumor necrosis factor alpha (TMFα) inhibitor" refers to any substance that reduces the activity of TNFα and comprises, without any limitations, substances that are used in combination therapy with GLP-2 or with both GLP-2 and an insulinotropic peptide, in which the combined therapy can prevent, improve, or treat bowel disease more effectively when than when used alone. Specifically, the TNFα inhibitor may be a substance that reduces TNFα activity by binding to TNFα or TNFα receptor, thereby ultimately blocking the interaction between TNFα and its receptor; or a substance that reduces TNFα activity by reducing TNFα production of cells, and these substances are not limited in their forms, such as compounds, nucleic acids, or peptides.

In one specific example, the TNFα inhibitor may be a soluble TNF receptor [for example, etanercept], an anti-TNFα antibody or a fragment thereof [for example, infliximab, adalimumab, certolizumab pegol, and golimumab], a compound [for example, thalidomide and derivatives thereof (for example, lenalidomide and pomalidomide); xanthine and derivatives thereof (for example, pentoxifylline); bupropion; 5-HT_{2A} agonists (for example, (R)-2,5-Dimethoxy-4-iodoamphetamine ((R)-DOI), TCB-2, lysergic acid diethylamide (LSD), lysergic acid 2,4-dimetylazetidide (LA-SS-Az))], or a combination thereof, but is not limited thereto. In addition to these substances, other substances known in the art to reduce TNFα activity may also be used as TNFα inhibitors in the present invention. Specifically, the TNFα inhibitor may be a soluble TNF receptor, anti-TNFα antibody or a fragment thereof, or a combination thereof, but is not limited thereto.

In the present invention, the insulinotropic peptide and/or GLP-2 may be in the form of a long-acting conjugate to which a biocompatible substance capable of increasing the *in vivo* half-life of each of the insulinotropic peptide and/or GLP-2 is bound, but is not limited thereto. The long-acting conjugate may exhibit enhanced persistence of efficacy compared to a derivative of insulinotropic peptide or GLP-2 to which a biocompatible substance (for example, an immunoglobulin Fc region) is not bound. In the present invention, a conjugate containing an insulinotropic peptide or GLP-2 with an increased half-life by binding of a biocompatible substance to the insulinotropic peptide or GLP-2 is referred to as an "insulinotropic peptide long-acting conjugate or long-acting conjugate of insulinotropic peptide" or a "GLP-2 long-acting conjugate or long-acting conjugate of GLP-2". In the present invention, a long-acting conjugate is used interchangeably with a conjugate.

In an embodiment,
(i) the insulinotropic peptide may be in the form of a long-acting conjugate to which a biocompatible substance capable of increasing *in vivo* half-life of the insulinotropic peptide is bound,
(ii) the GLP-2 may be in the form of a long-acting conjugate to which a biocompatible substance capable of increasing *in vivo* half-life of the GLP-2 is bound; or
(iii) each of the insulinotropic peptide and GLP-2 may be in the form of a long-acting conjugate to which a biocompatible substance capable of increasing in *vivo* half-life of each of the insulinotropic peptide and GLP-2 is bound.

### Meanwhile, these conjugates may be those which occur non-naturally.

In addition, the linkage between the insulinotropic peptide or GLP-2 and the biocompatible substance (for example, an immunoglobulin Fc region) in the long-acting conjugate may be a physical or chemical bond, or a non-covalent or covalent bond, and may specifically be a covalent bond, but is not limited thereto.

In addition, the method of linking the insulinotropic peptide or GLP-2 and the biocompatible substance (for example, an immunoglobulin Fc region) in the long-acting conjugate is not particularly limited, but the insulinotropic peptide or GLP-2 and the biocompatible substance (for example, an immunoglobulin Fc region) may be linked to each other through a linker.

In addition, Korean Patent Publication No. 10-2019-0037181 regarding a long-acting conjugate of GLP-2 is incorporated herein by reference.

In a specific embodiment, the long-acting conjugate of insulinotropic peptide or GLP-2 of the present invention may have a structure represented by the following Formula 1, but is not limited thereto.

[Formula 1] X-La-F

wherein,
X is an insulinotropic peptide or GLP-2;
L is a linker (for example, a linker comprising an ethylene glycol repeating unit);
a is 0 or a natural number, provided that each L is independent of each other when a is 2 or more;
F is a biocompatible substance capable of increasing the *in vivo* half-life of X (for example, an immunoglobulin Fc region); and
the "-" is a chemical bond (for example, a covalent bond).

More specifically, X and L, and L and F may be linked to each other by a covalent bond, and in particular, the conjugate may be a conjugate in which X, L, and F are linked to each other by a covalent bond in the order of Formula 1.

In addition, F may be directly linked to X (that is, a in Formula 1 is 0) or linked through a linker (L).

In the conjugate according to the present invention, F is a substance capable of increasing the half-life of X, that is, the insulinotropic peptide or GLP-2 according to the present invention, and corresponds to a constituent of a moiety constituting the conjugate of the present invention.

The F may be bonded to X by a covalent chemical bond or a non-covalent chemical bond, or F and X may be bonded to each other through L by a covalent chemical bond, a non-covalent chemical bond, or a combination thereof.

The F is a biocompatible substance capable of increasing the *in vivo* half-life of X, and may be selected from the group consisting of, for example, a high molecular weight polymer, a fatty acid, cholesterol, albumin and a fragment thereof, an albumin binding substance, a polymer of a repeating unit of a specific amino acid sequence, an antibody, an antibody fragment, an FcRn binding substance, *in vivo* connective tissue, a nucleotide, fibronectin, a transferrin, a saccharide, heparin, and elastin, but is not particularly limited thereto.

The elastin may be human tropoelastin, which is a water-soluble precursor, may be a polymer of some sequences or some repeating units thereof, and includes, for example, all elastin-like polypeptides, but is not particularly limited thereto.

Examples of the high molecular weight polymer may include a high molecular weight polymer selected from the group consisting of polyethylene glycol (PEG), polypropylene glycol, ethylene glycol-propylene glycol copolymer, polyoxyethylated polyol, polyvinyl alcohol, polysaccharides, polyvinyl ethyl ether, biodegradable polymers, lipid polymers, chitin, hyaluronic acid, oligonucleotides, and combinations thereof, and the polysaccharide may include dextran, but is not particularly limited thereto.

In the present specification, polyethylene glycol is a term encompassing all forms of ethylene glycol homopolymer, PEG copolymer, or monomethyl-substituted PEG polymer (mPEG), but is not particularly limited thereto.

In addition, the biocompatible substance includes poly-amino acids such as poly-lysine, poly-aspartic acid, and poly-glutamic acid, but is not limited thereto.

In addition, the fatty acid may be one having binding affinity with albumin *in vivo,* but is not particularly limited thereto.

As an example of the F, the F may be an FcRn-binding substance, and the FcRn-binding substance may be specifically an immunoglobulin Fc region, more specifically, an IgG Fc region, still more specifically, an aglycosylated IgG4 Fc region, but the F is not particularly limited thereto.

As a specific example of the present invention, the F (for example, an immunoglobulin Fc region) is a dimer composed of two polypeptide chains, and may have a structure in which one end of L is linked to only one polypeptide chain of the two polypeptide chains, but is not limited thereto.

In a specific embodiment, the long-acting conjugate of the present invention may be one in which an insulinotropic peptide or GLP-2 is linked to an immunoglobulin Fc region, but is not limited thereto.

In the present invention, the term "immunoglobulin Fc region" means a heavy chain constant region excluding the heavy chain and light chain variable regions of immunoglobulin. Specifically, the immunoglobulin Fc region may include a heavy chain constant region 2 (CH2) and/or a heavy chain constant region 3 (CH3) portion, and more specifically may further include a hinge region (meaning the whole or part of the hinge region). The immunoglobulin Fc region may be a constituent of a moiety of the conjugate of the present invention. Specifically, the immunoglobulin Fc region corresponds to F in Formula 1.

In the present specification, the Fc region not only includes the native sequence obtained from papain digestion of immunoglobulin, but also encompasses the derivatives, substitutions, and modifications thereof, for example, a sequence in which one or more amino acid residues in the native sequence are altered by deletion, insertion, non-conservative or conservative substitution, or a combination thereof to be different from the native sequence. The derivatives, substitutions, and modifications are premised on having the ability to bind to FcRn.

The F (for example, an immunoglobulin Fc region) has a structure in which two polypeptide chains are linked by a disulfide bond, and may have a structure in which the linkage is formed only through a nitrogen atom in one of the two chains, but is not limited thereto. The linkage through a nitrogen atom may be linked through reductive amination of the epsilon-amino atom of lysine or the N-terminal amino group.

The reductive amination reaction refers to a reaction in which an amine group or an amino group of a reactant reacts with an aldehyde (namely, a functional group capable of reductive amination) of another reactant to produce an amine and then an amine bond is formed by a reduction reaction, and is an organic synthesis reaction widely known in the art.

As a specific example, the immunoglobulin Fc region may be linked through its N-terminal nitrogen atom.

Such an immunoglobulin Fc region may include a hinge region in a heavy chain constant region, but is not limited thereto.

In the present invention, the immunoglobulin Fc region may include a specific hinge sequence at the N-terminus.

As used herein, the term "hinge sequence" refers to a site located in a heavy chain that forms a dimer of immunoglobulin Fc region through an interdisulfide bond.

In the present invention, the hinge sequence may be mutated to have only one cysteine residue by deletion of a part of the hinge sequence having the following amino acid sequence, but is not limited thereto:
Glu-Ser-Lys-Tyr-Gly-Pro-Pro-Cys-Pro-Ser-Cys-Pro (SEQ ID NO: 11).

The hinge sequence may include only one cysteine residue by deletion of a cysteine residue at position 8^{th} or 11^{th} in the hinge sequence of SEQ ID NO: 11. The hinge sequence of the present invention may consist of 3 to 12 amino acids including only one cysteine residue, but is not limited thereto. More specifically, the hinge sequence of the present invention may have the following sequences: Glu-Ser-Lys-Tyr-Gly-Pro-Pro-Pro-Ser-Cys-Pro (SEQ ID NO: 12), Glu-Ser-Lys-Tyr-Gly-Pro-Pro-Cys-Pro-Ser-Pro (SEQ ID NO: 12) No. 13), Glu-Ser-Lys-Tyr-Gly-Pro-Pro-Cys-Pro-Ser (SEQ ID NO: 14), Glu-Ser-Lys-Tyr-Gly-Pro-Pro-Cys-Pro-Pro (SEQ ID NO: 15), Lys-Tyr-Gly-Pro-Pro-Cys-Pro-Ser (SEQ ID NO: 16), Glu-Ser-Lys-Tyr-Gly-Pro-Pro-Cys (SEQ ID NO: 17), Glu-Lys-Tyr-Gly-Pro-Pro-Cys (SEQ ID NO: 18), Glu-Ser-Pro-Ser-Cys-Pro (SEQ ID NO: 19), Glu-Pro-Ser-Cys-Pro (SEQ ID NO: 20) , Pro-Ser-Cys-Pro (SEQ ID NO: 21), Glu-Ser-Lys-Tyr-Gly-Pro-Pro-Ser-Cys-Pro (SEQ ID NO: 22), Lys-Tyr-Gly-Pro-Pro-Pro-Ser-Cys-Pro (SEQ ID NO: 23), Glu-Ser-Lys-Tyr-Gly-Pro-Ser-Cys-Pro (SEQ ID NO: 24), Glu-Ser-Lys-Tyr-Gly-Pro-Pro-Cys (SEQ ID NO: 25), Lys-Tyr-Gly-Pro-Pro-Cys-Pro (SEQ ID NO: 26), Glu-Ser-Lys-Pro-Ser-Cys-Pro (SEQ ID NO: 27), Glu-Ser-Pro-Ser-Cys-Pro (SEQ ID NO: 28), Glu-Pro-Ser-Cys (SEQ ID NO: 29), and Ser -Cys-Pro (SEQ ID NO: 30).

More specifically, the hinge sequence may include the amino acid sequence of SEQ ID NO: 21 (Pro-Ser-Cys-Pro) or SEQ ID NO: 30 (Ser-Cys-Pro), but is not limited thereto.

In a more specific form of the long-acting conjugate of the present invention, the N-terminus of the immunoglobulin Fc region in the conjugate is proline, and the conjugate has the immunoglobulin Fc region linked to a linker through a nitrogen atom of the proline.

In a specific embodiment, the immunoglobulin Fc region may be in a dimeric form in which two chains of the immunoglobulin Fc region form a homodimer or a heterodimer due to the presence of a hinge sequence. The conjugate represented by Formula 1 of the present invention may be in a form in which one end of a linker is linked to one chain of the immunoglobulin Fc region of the dimer, but is not limited thereto.

As used herein, the term "N-terminus" refers to the amino terminus of a protein or polypeptide, and may include the final residue at the amino terminus, or up to 1, 2, 3, 4, 5, 6, 7, 8, 9, or 10 or more amino acids from the final residue of the terminus. The immunoglobulin Fc region of the present invention may include a hinge sequence at the N-terminus, but is not limited thereto.

In addition, the immunoglobulin Fc region of the present invention may be an extended Fc region comprising part or whole of heavy chain constant region 1 (CH1) and/or light chain constant region 1 (CL1) and excluding only the heavy and light chain variable regions of immunoglobulin provided that it has substantially equivalent or improved effect as that of the native type. In addition, the immunoglobulin Fc region may be a region in which part of a fairly long amino acid sequence corresponding to CH2 and/or CH3 is removed.

For example, the immunoglobulin Fc region of the present invention may be 1) CH1 domain, CH2 domain, CH3 domain, and CH4 domain, 2) CH1 domain and CH2 domain, 3) CH1 domain and CH3 domain, 4) CH2 domain and CH3 domain, 5) a combination of one or two or more domains among CH1 domain, CH2 domain, CH3 domain, and CH4 domain with an immunoglobulin hinge region (or part of the hinge region), or 6) a dimer of each domain of a heavy chain constant region and a light chain constant region, but is not limited thereto.

In addition, as an embodiment of the long-acting conjugate of the present invention, the immunoglobulin Fc region may be in a dimeric form, and one molecule of X may be covalently linked to one Fc region in a dimeric form. In particular, the immunoglobulin Fc and X may be linked to each other through the same linker. Meanwhile, it is also possible to bind two X molecules to one Fc region in a dimeric form symmetrically. In particular, the immunoglobulin Fc and X may be linked to each other through a linker. However, the immunoglobulin Fc region is not limited to the examples described above.

In addition, the immunoglobulin Fc region of the present invention includes not only a native amino acid sequence but also a sequence derivative thereof. An amino acid sequence derivative refers to a sequence that differs from a native amino acid sequence by one or more amino acid residues through deletion, insertion, non-conservative or conservative substitution, or a combination thereof.

For example, in IgG Fc, amino acid residues at positions 214 to 238, 297 to 299, 318 to 322, or 327 to 331, which are known to be important for binding, may be used as suitable sites for modification.

In addition, it is possible to obtain various types of derivatives by removing a site capable of forming a disulfide bond, removing several amino acids at the N-terminus of the native Fc, or adding a methionine residue to the N-terminus of the native Fc, and the like. Further, in order to eliminate the effector function, a complement binding site, for example, a C1q binding site, may be removed, or an antibody dependent cell mediated cytotoxicity (ADCC) site may be removed. Such techniques for preparing sequence derivatives of immunoglobulin Fc region are disclosed in International Patent Application Publication No. WO 97/34631, International Patent Application Publication No. 96/32478, and the like.

Amino acid exchanges in proteins and peptides that do not substantially alter the activity of the molecule are known in the art (H. Neurath, R.L. Hill, The Proteins, Academic Press, New York, 1979). The most commonly occurring exchange is between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, amino acids may be modified by phosphorylation, sulfation, acrylation, glycosylation, methylation, farnesylation, acetylation, amidation, and the like.

The above-described Fc derivative exhibit biological activity equivalent to that of the Fc region of the present invention and may have enhanced the structural stability of the Fc region against heat and pH.

In addition, such an Fc region may be obtained from a native type isolated in *vivo* from animals such as humans, cows, goats, pigs, mice, rabbits, hamsters, rats, guinea pigs, or the like, or the Fc region may be a recombinant obtained from a transformed animal cell or microorganism, or a derivative thereof. In particular, the method of obtaining an Fc region from a native type may be a method of obtaining the Fc region by isolating the whole immunoglobulin from a living body of a human or animal and then treating the immunoglobulin with a protease. The immunoglobulin is cleaved into Fab and Fc when treated with papain, and the immunoglobulin is cleaved into pF'c and F(ab)2 when treated with pepsin. Fc or pF'c may be isolated from this by size-exclusion chromatography or the like. In a more specific embodiment, the Fc region is a recombinant immunoglobulin Fc region in which a human-derived Fc region is obtained from a microorganism.

In addition, the immunoglobulin Fc region may be a native type sugar chain, an increased sugar chain compared to the native type, a decreased sugar chain compared to the natural type, or a form in which the sugar chain is removed. Conventional methods such as chemical methods, enzymatic methods, and genetic engineering methods using microorganisms may be used to increase, decrease, or remove immunoglobulin Fc sugar chains. In particular, the immunoglobulin Fc region in which the sugar chain is removed from Fc has significantly reduced ability to bind to complement (c1q), has decreased or eliminated antibody dependent cytotoxicity or complement-dependent cytotoxicity, and thus does not induce unnecessary immune responses *in vivo.* In this regard, a form more suitable for its original purpose as a drug carrier is a deglycosylated or aglycosylated immunoglobulin Fc region.

In the present invention, the term "deglycosylation" refers to an Fc region in which sugar is removed by an enzyme, and the term "aglycosylation" means an Fc region that is produced by a prokaryotic animal, in a more specific embodiment, E. coli, and is not glycosylated.

Meanwhile, the immunoglobulin Fc region may be of human origin or animal origin such as cow, goat, pig, mouse, rabbit, hamster, rat, guinea pig, or the like, and in a more specific embodiment, is of human origin.

In addition, the immunoglobulin Fc region may be an Fc region derived from IgG, IgA, IgD, IgE, or IgM, a combination thereof, or a hybrid thereof. In a more specific embodiment, the immunoglobulin Fc region is derived from IgG or IgM, which is most abundant in human blood, and in a still more specific embodiment, the immunoglobulin Fc region is derived from an IgG known to increase the half-life of ligand binding proteins. In a yet still more specific embodiment, the immunoglobulin Fc region is an IgG4 Fc region, and in the most specific embodiment, it is an aglycosylated Fc region derived from human IgG4, but is not limited thereto.

In addition, as an embodiment, the immunoglobulin Fc region is a fragment of human IgG4 Fc and may be a homodimer in which two monomers are linked by a disulfide bond (inter-chain form) between cysteines, which are amino acid at position 3 of the respective monomers, and in particular, the homodimer has/may have a disulfide bond between cysteines at positions 35 and 95 and a disulfide bond between cysteines at positions 141 and 199, that is, two disulfide bonds (intra-chain form) in each monomer.

The number of amino acids in each monomer may be 221, and the amino acids forming a homodimer may be a total of 442 amino acids, but are not limited thereto. Specifically, the immunoglobulin Fc region may be a homodimer including the amino acid sequence of SEQ ID NO: 35 (consisting of 442 amino acids) (in particular, two monomers having the amino acid sequence of SEQ ID NO: 31, which consist of 221 amino acids, may form a homodimer by a disulfide bond between cysteines, which are amino acids at position 3 of the respective monomers, and the monomers of the homodimer may each independently form an intramolecular disulfide bond between cysteines at positions 35 and 95 and an intramolecular disulfide bond between cysteines at positions 141 and 199), but is not limited thereto.

Meanwhile, in the present invention, the term "combination", which is related to the immunoglobulin Fc region, means that a polypeptide encoding a single-chain immunoglobulin Fc region of the same origin forms a bond with a single-chain polypeptide of a different origin when a dimer or a multimer is formed. In other words, a dimer or a multimer can be prepared from two or more Fc regions selected from the group consisting of IgG Fc, IgA Fc, IgM Fc, IgD Fc, and IgE Fc regions.

In the present invention, the term "hybrid" means that sequences corresponding to immunoglobulin Fc regions of two or more different origins are present in a single-chain immunoglobulin constant region. In the present invention, various kinds of hybrids are possible. In other words, hybridization of domains can consist of one to four domains from the group consisting of CH1, CH2, CH3, and CH4 of IgG Fc, IgM Fc, IgA Fc, IgE Fc, and IgD Fc, and may include a hinge.

Meanwhile, IgG can also be divided into subclasses of IgG1, IgG2, IgG3, and IgG4, and in the present invention, combinations thereof or hybridizations thereof may be used. Specifically, the immunoglobulin Fc region is IgG2 and IgG4 subclasses, and, most specifically, an Fc region of IgG4 that has almost no effector function such as complement dependent cytotoxicity (CDC).

The above-described conjugate may exhibit enhanced persistence of efficacy compared to that of native insulinotropic peptide, native GLP-2, or X not modified with F, and such a conjugate comprises not only the above-described forms but also all forms encapsulated in biodegradable nanoparticles and the like, but is not limited thereto.

In Formula 1, the linker L may be a peptide linker or a non-peptide linker (for example, a linker comprising an ethylene glycol repeating unit).

When the L is a peptide linker, the peptide linker may contain one or more amino acids, for example, from 1 to 1000 amino acids, but is not particularly limited thereto. In the present invention, various known peptide linkers may be used to link F and X, and examples include [GS]x linker, [GGGS]x linker, and [GGGGS]x linker, where x may be a natural number of 1 or more. However, the peptide linker is not limited to these examples.

In the present invention, the term "non-peptide linker" includes a biocompatible polymer in which two or more repeating units are bonded. The repeating units are linked to each other by any covalent bond that is not a peptide bond. The non-peptide linker may be a constituent of a moiety of the conjugate of the present invention and corresponds to L in Formula 1.

The non-peptide linker used in the present invention may be any polymer resistant to proteolytic enzymes *in vivo* without limitation.

In the present invention, the non-peptide linker may be used interchangeably with a non-peptide polymer.

In addition, although not particularly limited, the non-peptide linker may be selected from the group consisting of polyethylene glycol, polypropylene glycol, a copolymer of ethylene glycol and propylene glycol, polyoxyethylated polyol, polyvinyl alcohol, polysaccharides (for example, dextran), polyvinyl ethyl ether, biodegradable polymers such as polylactic acid (PLA) and polylactic-glycolic acid (PLGA), lipid polymers, chitin, hyaluronic acid, oligonucleotides, and combinations thereof.

In addition, the non-peptide linker that can be used in the present invention may be any polymer that is resistant to proteases *in vivo* without limitation, but specifically, the molecular weight of the non-peptide polymer is in a range of more than 0 kDa to about 100 kDa, about 1 kDa to about 100 kDa, specifically in a range of about 1 kDa to about 50 kDa, about 1 kDa to about 20 kDa, about 1 kDa to about 10 kDa, or about 3.4 kDa to about 10 kDa, but is not limited thereto.

Although not particularly limited, the non-peptide linker may be a linker comprising an ethylene glycol repeating unit, for example, polyethylene glycol, and derivatives thereof already known in the art and derivatives that can be easily prepared at the level of skill in the art are also included in the scope of the present invention.

The repeating unit of the non-peptide linker may be an ethylene glycol repeating unit. Specifically, the non-peptide linker may comprise an ethylene glycol repeating unit while comprising a functional group used in the preparation of conjugate at its terminus. The long-acting conjugate according to the present invention may be in a form in which X and F are linked through the functional group, but is not limited thereto. In the present invention, the non-peptide linker may contain two or three or more functional groups, and the respective functional groups may be the same as or different from each other, but are not limited thereto.

Specifically, the linker may comprise a repeating unit represented by the following Formula 4. An example thereof includes polyethylene glycol (PEG), but is not limited thereto: 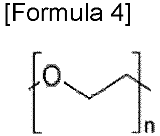 wherein, n = 10 to 2400, n = 10 to 480, or n = 50 to 250, but is not limited thereto.

In the long-acting conjugate, the PEG moiety may include not only a - (CH₂CH₂O)ₙ- structure but also an oxygen atom interposed between the linking element and -(CH₂CH₂O)ₙ-, but is not limited thereto.

Further, in a specific embodiment, the conjugate may be a structure in which an insulinotropic peptide or GLP-2, and an immunoglobulin Fc region (F) are covalently linked through a linker containing an ethylene glycol repeating unit, but is not limited thereto.

The term "polyethylene glycol" encompasses all forms of ethylene glycol homopolymer, PEG copolymer, and monomethyl-substituted PEG polymer (mPEG), but is not particularly limited thereto.

In an embodiment, the ethylene glycol repeating unit may be represented by, for example, [OCH₂CH₂]ₙ, in which the n value is a natural number and may be determined such that the average molecular weight of the [OCH₂CH₂]ₙ moiety in the peptide conjugate, for example, the number average molecular weight, is more than 0 kDa to about 100 kDa, but is not limited thereto. In another example, the n value is a natural number and the average molecular weight of the [OCH₂CH₂]ₙ moiety in the peptide conjugate, for example, the number average molecular weight, may be about 1 kDa to about 100 kDa, about 1 kDa to about 80 kDa, about 1 kDa to about 50 kDa, about 1 kDa to about 30 kDa, about 1 kDa to about 25 kDa, about 1 kDa to about 20 kDa, about 1 kDa to about 15 kDa, about 1 kDa to about 13 kDa, about 1 kDa to about 11 kDa, about 1 kDa to about 10 kDa, about 1 kDa to about 8 kDa, about 1 kDa to about 5 kDa, about 1 kDa to about 3.4 kDa, about 3 kDa to about 30 kDa, about 3 kDa to about 27 kDa, about 3 kDa to about 25 kDa, about 3 kDa to about 22 kDa, about 3 kDa to about 20 kDa, about 3 kDa to about 18 kDa, about 3 kDa to about 16 kDa, about 3 kDa to about 15 kDa, about 3 kDa to about 13 kDa, about 3 kDa to about 11 kDa, about 3 kDa to about 10 kDa, about 3 kDa to about 8 kDa, about 3 kDa to about 5 kDa, about 3 kDa to about 3.4 kDa, about 8 kDa to about 30 kDa, about 8 kDa to about 27 kDa, about 8 kDa to about 25 kDa, about 8 kDa to about 22 kDa, about 8 kDa to about 20 kDa, about 8 kDa to about 18 kDa, about 8 kDa to about 16 kDa, about 8 kDa to about 15 kDa, about 8 kDa to about 13 kDa, about 8 kDa to about 11 kDa, about 8 kDa to about 10 kDa, about 9 kDa to about 15 kDa, about 9 kDa to about 14 kDa, about 9 kDa to about 13 kDa, about 9 kDa to about 12 kDa, about 9 kDa to about 11 kDa, about 9.5 kDa to about 10.5 kDa, about 3.4 kDa, or about 10 kDa, but is not limited thereto.

As used herein, the term "about" is a range including all of ±0.5, ±0.4, ±0.3, ±0.2, ±0.1, and the like, and includes all numerical values equivalent to or similar to the numerical values following the term "about", but is not limited thereto.

In addition, the non-peptide linker of the present invention bound to the immunoglobulin Fc region may be not only a type of polymer but also a combination of different types of polymers.

In a specific embodiment, both ends of the non-peptide linker may bind to a thiol group, an amino group, or a hydroxyl group of an immunoglobulin Fc region and a thiol group, an amino group, an azido group, or a hydroxyl group of GLP-2, but are not limited thereto.

Specifically, the non-peptide linker may include reactive groups capable of binding to immunoglobulin Fc and an insulinotropic peptide or GLP-2 at both ends, respectively, specifically, reactive groups capable of binding to a thiol group of cysteine; an amino group located at the N-terminus, lysine, arginine, glutamine and/or histidine; and/or a hydroxyl group located at the C-terminus in the immunoglobulin Fc region and capable of binding to a thiol group of cysteine; an amino group of lysine, arginine, glutamine and/or histidine; an azido group of azidolysine; and/or a hydroxyl group in GLP-2, but is not limited thereto.

More specifically, the reactive group of the non-peptide polymer may be one or more selected from the group consisting of an aldehyde group, a maleimide group, and a succinimide derivative, but is not limited thereto.

In the above, examples of the aldehyde group include a propionaldehyde group or a butyl aldehyde group, but are not limited thereto.

In the above, the succinimide derivative may be succinimidyl carboxymethyl, succinimidyl valerate, succinimidyl methylbutanoate, succinimidyl methylpropionate, succinimidyl butanoate, succinimidyl propionate, N-hydroxysuccinimide, hydroxy succinimidyl or succinimidyl carbonate, but is not limited thereto.

The non-peptide linker may be converted into a non-peptide polymer linkage by linking a biocompatible substance (for example, immunoglobulin Fc) with an insulinotropic peptide or GLP-2 through a reactive group as described above.

In addition, the end products produced through reductive alkylation by an aldehyde bond are more stable than those linked by an amide bond. The aldehyde reactive group selectively reacts to the N-terminus at a low pH and can form a covalent bond with a lysine residue at a high pH, for example, pH 9.0.

Terminal reactive groups of the non-peptide linker of the present invention may be the same as or different from each other. The non-peptide linker may have an aldehyde reactive group at the ends, and additionally, the non-peptide linker may have an aldehyde group and a maleimide reactive group at each end or an aldehyde group and a succinimide reactive group at each end, but is not limited thereto.

For example, the non-peptide linker may have a maleimide group at one end and an aldehyde group, propionaldehyde group, or butyl aldehyde group at the other end. In another example, the non-peptide linker may have a succinimidyl group at one end and a propionaldehyde group or a butyl aldehyde group at the other end.

In a case where polyethylene glycol having a hydroxyl group at the propionic end is used as a non-peptide linker, the conjugate of the present invention can be prepared by activating the hydroxyl group into various reactive groups through known chemical reactions or using commercially available polyethylene glycol having a modified reactive group.

In a specific embodiment, the reactive group of the non-peptide linker may be linked to a cysteine residue in an insulinotropic peptide or GLP-2, more specifically, the -SH group of the cysteine, but is not limited thereto.

If maleimide-PEG-aldehyde is used, the maleimide group may be linked to the -SH group in the insulinotropic peptide or GLP-2 by a thioether bond, and the aldehyde group may be linked to the -NH₂ group in an immunoglobulin Fc through a reductive alkylation reaction, but the non-peptide linker is not limited thereto, and this corresponds to one example.

Through such reductive alkylation, the N-terminal amino group in the immunoglobulin Fc region may be linked to an oxygen atom located at one end of PEG through a linker functional group having a structure of -CH₂CH₂CH₂- to form a structure such as -PEG-O-CH₂CH₂CH₂NH-immunoglobulin Fc, and a structure in which one end of PEG is linked to a sulfur atom located in GLP-2 or at cysteine of GLP-2 may be formed by a thioether bond. The above-mentioned thioether bond may include a structure of

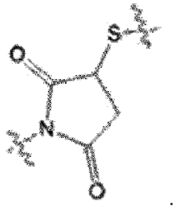

However, the non-peptide linker is not particularly limited to the examples described above, and this only corresponds to an example.

Further, in the conjugate, the reactive group of the non-peptide linker may be linked to -NH₂ located at the N-terminus of the immunoglobulin Fc region, but this corresponds to one example.

Further, in the conjugate, an insulinotropic peptide or GLP-2 may be linked to a non-peptide linker having a reactive group through the C-terminus, but this corresponds to one example.

In the present invention, the term "C-terminus" refers to the carboxy-terminus of a peptide, and refers to a position capable of binding to a non-peptide polymer for the purpose of the present invention. For example, although the C-terminus is not limited, it may include the terminal amino acid residue of the C-terminus as well as amino acid residues around the C-terminus, specifically may include the first to the twentieth amino acid residues from the terminus.

In an embodiment, the conjugate represented by Formula 1 may have a structure represented by the following Formula 2 or 3.

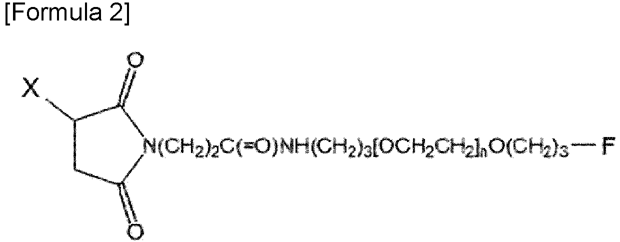

[Formula 3] X-(CH₂)₃[OCH₂CH₂}ₙO(CH₂)₃-_{F}

In Formula 2 or 3, X may be the above-described peptide in Formula 1;
F may be an immunoglobulin Fc fragment; and
n may be a natural number. In particular, the description of n is as described above.

In an embodiment, the long-acting conjugate represented by Formula 2 may have a structure in which X of an insulinotropic peptide or GLP-2 and F of an immunoglobulin Fc region are covalently linked via an ethylene glycol repeating unit, in which X may be linked to a succinimide ring in Formula 2 and F to an oxypropylene group in Formula 2. In addition, the long-acting conjugate represented by Formula 3 may have a structure in which X of an insulinotropic peptide or GLP-2 and F of an immunoglobulin Fc region are covalently linked via an ethylene glycol repeating unit, and X may be linked to an oxypropylene group in Formula 3 and F to another oxypropylene group in Formula 3.

In Formula 2 or 3, the value of n may be determined such that the average molecular weight of the [OCH₂CH₂]ₙ moiety in the peptide conjugate, for example, the number average molecular weight, is 1 kDa to 100 kDa, or 1 kDa to 20 kDa, or 10 kDa, but is not limited thereto.

In an embodiment, the succinimide ring in Formula 2 or the site to which X is linked on the succinimide ring of Formula 2 may be the sulfur atom of the C-terminal cysteine of X. In addition, the oxypropylene group in Formula 3 or the site to which X is linked on the oxypropylene group of Formula 3 may be the sulfur atom of the C-terminal cysteine of X.

The site in F linked to the oxypropylene group in Formula 2 or Formula 3 is not particularly limited. In an embodiment of the present invention, the site of F linked to the oxypropylene group may be N-terminal nitrogen or a nitrogen atom of a residue inside F (for example, the epsilon-nitrogen of lysine). In a specific embodiment of the present invention, the site where F is linked to the oxypropylene group in Formula 2 or Formula 3 may be the N-terminal proline of F, but is not limited thereto.

Meanwhile, the insulinotropic peptide, GLP-2, a long-acting conjugate thereof, or a TNFα inhibitor according to the present invention includes all forms of itself, a salt thereof (for example, a pharmaceutically acceptable salt), or a solvate thereof.

In addition, the insulinotropic peptide, GLP-2, a long-acting conjugate thereof, or a TNFα inhibitor may be in any pharmaceutically acceptable form.

The kind of salt is not particularly limited. However, the salt is preferably in a form that is safe and effective for a subject, for example, a mammal, but is not particularly limited thereto.

As used herein, the term "pharmaceutically acceptable salt" includes salts derived from pharmaceutically acceptable inorganic acids, organic acids, or bases. Examples of suitable acids include hydrochloric acid, hydrobromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, and the like. Salts derived from suitable bases may comprise alkali metals such as sodium and potassium, or alkaline earth metals such as magnesium, and ammonium.

As used herein, the term "solvate" refers to a complex formed from a peptide, compound, or salt thereof according to the present invention and a solvent molecule.

The native insulinotropic peptide and modified insulinotropic peptide, GLP-2 and GLP-2 derivative, and TNFα inhibitor of the present invention may be synthesized through a solid phase synthesis method, can also be produced by a recombinant method, and may be prepared by requesting commercially, and commercially available products may be used.

In another embodiment, the combination, pharmaceutical composition, or pharmaceutical kit of the present invention may comprise:
(i) GLP-2 and an insulinotropic peptide,
   GLP-2 and a TNFα inhibitor, or
   GLP-2, an insulinotropic peptide, and a TNFα inhibitor;
(ii) GLP-2, and an insulinotropic peptide long-acting conjugate bound to a biocompatible substance capable of increasing its *in vivo* half-life, or
   GLP-2, the insulinotropic peptide long-acting conjugate, and a TNFα inhibitor;
(iii) a GLP-2 long-acting conjugate bound to a biocompatible substance capable of increasing its *in vivo* half-life, and an insulinotropic peptide,
   the GLP-2 long-acting conjugate and a TNFα inhibitor, or
   the GLP-2 long-acting conjugate, an insulinotropic peptide, and a TNFα inhibitor; or
(iv) a GLP-2 long-acting conjugate bound to a biocompatible substance capable of increasing its *in vivo* half-life, and an insulinotropic peptide long-acting conjugate bound to a biocompatible substance capable of increasing its *in vivo* half-life, or
   the GLP-2 long-acting conjugate, the insulinotropic peptide long-acting conjugate, and a TNFα inhibitor.

The combination, pharmaceutical composition, or pharmaceutical kit of the present invention can be used to prevent, improve, or treat bowel disease

As used herein, the term "prevention" refers to any action that inhibits or delays the onset of a target disease, for example, bowel disease, by administration of a combination comprising GLP-2, and an insulinotropic peptide, a TNFα inhibitor, or both; or a composition comprising GLP-2, and an insulinotropic peptide, a TNFα inhibitor, or both. The term "treatment" refers to any action that improves or benefits the symptoms of a target disease, for example, bowel disease, by administration of a combination comprising GLP-2, and an insulinotropic peptide, a TNFα inhibitor, or both; or a composition comprising GLP-2, and an insulinotropic peptide, a TNFα inhibitor, or both. The term "improvement" refers to any action that at least diminishes a parameter associated with the condition being treated by the administration of a combination or composition of the present invention, for example, the severity of a symptom.

As used herein, the term "administration" refers to the introduction of a given substance to a patient by any suitable method, and while the route of administration of the composition is not particularly limited, it may be any general route through which the GLP-2, insulinotropic peptide, and TNFα inhibitor can reach the target *in vivo.* The administration may be, for example, intraperitoneal administration, intravenous administration, intramuscular administration, subcutaneous administration, intradermal administration, oral administration, topical administration, intranasal administration, intrapulmonary administration, intrarectal administration, or the like.

In the method of the present invention, the GLP-2, insulinotropic peptide, and TNFα inhibitor may be administered via the same route of administration or via different routes of administration, and the routes of administration for the drugs administered in combination may be independent of one another.

The GLP-2 may be used in combination with an insulinotropic peptide, a TNFα inhibitor, or both to prevent or treat bowel disease.

The bowel disease may be at least one selected from the group consisting of irritable bowel disease, enteritis, inflammatory bowel disease, enterocolitis, colitis, pancreatitis, ileitis, bowel atrophy, and bowel damage, but bowel disease prevented, improved, or treated by the composition of the present invention is included without limitation.

In a specific example, the bowel disease may be inflammatory bowel disease. The inflammatory bowel disease (IBD) refers to an inflammatory disease characterized by inflammation or ulcers in the gastrointestinal tract, and may exhibit symptoms such as increased expression of inflammatory cytokines, weight loss, shortened colon length, abdominal pain, fever, diarrhea, and/or hematochezia. These symptoms may worsen and improve repeatedly, but are not limited thereto. Specifically, the inflammatory bowel disease may be at least one selected from the group consisting of ulcerative colitis, Crohn's disease, and Behcet's disease, but is not limited thereto.

The combination or composition according to the present invention may prevent, improve, or treat bowel disease by exhibiting one or more effects among inhibition of M1 polarization in monocytes, inhibition of macrophage differentiation, and inhibition of monocyte migration, but is not limited thereto.

When administered to a subject, the combination or composition according to the present invention prevent, improve, or treat bowel disease by causing at least one effect among weight gain, an increase in small intestine length, a decrease in gastrointestinal motility, and an increase in nutrient absorption, but is not limited thereto.

The pharmaceutical composition of the present invention may further comprise a pharmaceutically acceptable carrier, excipient, or diluent. Such a pharmaceutically acceptable carrier, excipient, or diluent may be that which occur non-naturally.

As used herein, the term "pharmaceutically acceptable" means having a sufficient amount to exhibit a therapeutic effect and not causing side effects, and may be easily determined by those skilled in the art according to factors well known in the medical field, such as the type of disease, age, weight, health, and sex of patient, drug sensitivity of patient, route of administration, method of administration, number of administration, treatment duration, and drugs combined or used concurrently. The pharmaceutical composition of the present invention may further comprise a pharmaceutically acceptable excipient. While the excipient is not particularly limited, binders, lubricants, disintegrants, solubilizers, dispersants, stabilizers, suspending agents, pigments, flavors, and the like may be used for oral administration; buffers, preservatives, soothing agents, solubilizers, isotonic agents, stabilizers, and the like may be used in mixture for injection; and bases, excipients, lubricants, preservatives, and the like may be used for topical administration.

The formulation of the composition of the present invention may be variously prepared by combining the composition with pharmaceutically acceptable excipients as described above. For example, the composition may be prepared in the form of tablets, troches, capsules, elixirs, suspensions, syrups, wafers, and the like for oral administration, and the composition may be prepared in unit dosage ampoules or multiple dose forms for injection. In addition, the composition may be formulated into solutions, suspensions, tablets, pills, capsules, sustained-release preparations, and the like.

Meanwhile, examples of carriers, excipients, and diluents suitable for preparations include lactose, dextrose, sucrose, sorbitol, mannitol, xylitol, erythritol, maltitol, starch, acacia, alginates, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, mineral oil or the like may be used. In addition, fillers, anti-coagulants, lubricants, wetting agents, flavoring agents, preservatives, and the like may be further included.

In addition, the pharmaceutical composition of the present invention may have any one formulation selected from the group consisting of tablets, pills, powders, granules, capsules, suspensions, solutions for internal use, emulsions, syrups, sterilized aqueous solutions, non-aqueous solvents, lyophilized preparations, and suppositories.

In addition, the composition may be formulated into a preparation in a unit dosage form suitable for administration into the body of a patient, specifically, a preparation useful for the administration of a protein drug, according to a conventional method in the pharmaceutical field and administered by an oral administration route or a parenteral administration route including a cutaneous, intravenous, intramuscular, intraarterial, intramedullary, intrathecal, intraventricular, pulmonary, transdermal, subcutaneous, intraperitoneal, intranasal, intragastric, topical, sublingual, intravaginal or rectal route using an administration method commonly used in the art, but is not limited thereto.

In addition, the conjugate may be used in combination with various pharmaceutically acceptable carriers such as physiological saline or organic solvents, and carbohydrates such as glucose, sucrose, or dextran, antioxidants such as ascorbic acid or glutathione, chelating agents, low molecular weight proteins, other stabilizers, or the like may be used as agents to increase stability or absorption.

The dose and number of administrations of the pharmaceutical composition of the present invention are determined according to the drug type of an active ingredient together with various related factors such as the target disease, route of administration, age, sex and weight of patient, and severity of disease. Specifically, the composition of the present invention may comprise the insulinotropic peptide, GLP-2, or TNFα inhibitor in a pharmaceutically effective amount, but is not limited thereto.

The inclusion of the insulinotropic peptide, GLP-2, or TNFα inhibitor in a pharmaceutically effective amount refers to an amount sufficient to achieve the desired pharmacological activity from the insulinotropic peptide, GLP-2, and/or TNFα inhibitor, and may refer to a pharmaceutically acceptable amount that does not cause or cause insignificant toxicity or side effects in the administered subject, but is not limited thereto. Such a pharmaceutically effective amount may be determined by comprehensively considering the number of administrations, patient, formulation, and the like.

Although not particularly limited, the pharmaceutical composition of the present invention may comprise the ingredient (active ingredient) in an amount of 0.01 w/v% to 99 w/v%.

The total effective amount of the composition of the present invention may be administered to a patient in a single dose or by a fractionated treatment protocol in which multiple doses are administered over a long period of time. The pharmaceutical composition of the present invention may comprise the active ingredient at different contents according to the severity of the disease. Specifically, a preferred total dose of the peptide or conjugate of the present invention may be about 0.0001 mg to 500 mg per 1 kg of the weight of patient per day, but is not limited thereto. However, the effective dose of the conjugate for a patient is determined in consideration of various factors, such as the age, weight, health condition, sex, diet and excretion rate, and the severity of the disease of the patient, as well as the route of administration of the pharmaceutical composition and the number of treatments. Accordingly, those skilled in the art will be able to determine an appropriate effective dose according to the specific use of the composition of the present invention considering the above. The pharmaceutical composition according to the present invention is not particularly limited in its formulation, administration route, and administration method as long as it exhibits the effect of the present invention.

The pharmaceutical composition of the present invention has excellent *in vivo* persistence and potency so that the number and frequency of administration of the pharmaceutical preparation of the present invention can be significantly decreased, but is not limited thereto.

Another aspect embodying the present invention provides a food composition for preventing, improving, or treating bowel disease comprising the GLP-2, in which the food composition is administered in combination with an insulinotropic peptide, a TNFα inhibitor, or both.

The insulinotropic peptide, GLP-2, TNFα inhibitor, prevention, treatment, improvement, and bowel disease are as described above.

The food composition may be used as a health functional food. In a case where the composition of the present invention is used as a food supplement additive, the insulinotropic peptide, GLP-2, a long-acting conjugate comprising the same, TNFα inhibitor, or a combination thereof may be added as-is or used in combination with other foods or food ingredients, and may be appropriately used according to conventional methods. The amount of an active ingredient used in combination may be appropriately determined depending on the purpose of use (prevention, health, or therapeutic treatment).

As used herein, the term "health functional food" refers to food manufactured and processed using specific ingredients as raw materials or by methods such as extraction, concentration, purification, and mixing of specific ingredients contained in food materials for the purpose of health supplementation, and refers to food designed and processed so as to fully exert the bioregulatory functions such as biodefense, regulation of biorhythm, prevention of disease, and recovery from disease with respect to a living body by the above ingredients. The composition for health food can perform functions related to prevention of disease, recovery from disease, and the like.

Another aspect embodying the present invention provides a method for preventing, improving, or treating bowel disease, including administering GLP-2, and an insulinotropic peptide, a TNFα inhibitor, or both to a subject in need thereof.

In another embodiment, the method may be a method for preventing, improving, or treating bowel disease, including administering and/or using the combination, pharmaceutical composition, or pharmaceutical kit of the present invention in a subject in need thereof.

In another embodiment, the method is a method for preventing, improving, or treating bowel disease, including administering and/or using a composition comprising GLP-2 in a pharmaceutically effective amount in combination with a composition comprising an insulinotropic peptide in a pharmaceutically effective amount, a composition comprising a TNFα inhibitor in a pharmaceutically effective amount, or both in a subject in need thereof.

The insulinotropic peptide, GLP-2, TNFα inhibitor, use in combination, prevention, treatment, improvement, bowel disease, combination, pharmaceutical composition, and pharmaceutical kit are as described above.

In the present invention, the subject is a subject suspected of having bowel disease, in which the subject suspected of bowel disease refers to mammals, including mice, livestock, as well as humans, that have developed or may develop the disease. However, subject that can be treated by GLP-2 in combination with an insulinotropic peptide, a TNFα inhibitor, or both of the present invention are included without limitation. In addition, by administering GLP-2 in combination with an insulinotropic peptide, a TNFα inhibitor, or both of the present invention to a subject suspected of bowel disease, the subject can be effectively treated. Bowel disease is as described above.

The method of the present invention may include administering a combination or pharmaceutical composition comprising GLP-2, and an insulinotropic peptide, a TNFα inhibitor, or both, in a pharmaceutically effective amount. The method according to the present invention may be administering GLP-2, and an insulinotropic peptide, a TNFα inhibitor, or both as a single preparation or as separate preparations administered simultaneously, individually, sequentially, or in reverse order, but the method is not limited thereto.

The appropriate total daily amount may be determined by a treating physician within the scope of sound medical judgment, and may be administered once or in a manner divided into several times. However, for the purpose of the present invention, the specific therapeutically effective amount for a particular patient is preferably applied differently based on various factors such as the type and degree of the desired response, in some cases, whether other agents are being used in combination, as well as the specific composition, the age, weight, general health condition, sex, and diet of patient, administration time, route of administration and secretion rate of the composition, treatment duration, and drugs used together with or simultaneously with the specific composition; and similar factors well known in the field of medicine.

Another aspect embodying the present invention is the use of the combination, pharmaceutical composition, or pharmaceutical kit for preventing, improving, or treating bowel disease and/or use of the combination, pharmaceutical composition, or pharmaceutical kit for the preparation of a medicament for preventing, improving, or treating bowel disease. The prevention, improvement, treatment ,bowel disease, combination, pharmaceutical composition, and pharmaceutical kit are as described above.

Hereinafter, the present invention will be described in more detail with reference to the following Examples. However, the following Examples are only for exemplifying the present invention, and the scope of the present invention is not limited thereto.

### Example 1: Preparation of long-acting GLP-1 derivative conjugate

### 1-1. Preparation of CA-exendin-4-PEG conjugate

A PEGylated peptide conjugate (mono-PEGylated CA-exendin-4) was prepared by a covalent bond between the amine group of the 27th lysine residue in CA-exendin-4 (N-[2-(1H-imidazol-5-yl)acetyl]-exendin-4, Hanmi Fine Chemical, Korea) peptide and polyethylene glycol having an aldehyde (ALD) group at both ends (3.4 kDa, ALD(2) PEG, Hanmi Fine Chemical, Korea). Specifically, the reaction was conducted at a molar ratio of CA-exendin-4 : ALD(2) PEG of 1:5 to 1:15, a concentration of CA-exendin-4 of 6 mg/mL to 12 mg/mL, and a temperature of 4°C to 10°C to room temperature for about 4 to 12 hours. At this time, the reaction was conducted in 0.1 M HEPES with a pH of 7.0 to 8.5 and about 45% isopropanol, and sodium cyanoborohydride (NaCNBH3) at a concentration of 2 mM to 50 mM was added for the reaction. The reaction mixture was purified using Source 15S (Cytiva, USA) column using a buffer comprising sodium citrate (pH of 2.0 to 3.5) and about 45% ethanol and potassium chloride concentration gradient.

### 1-2. Preparation of CA-exendin-4-PEG-Fc conjugate

In order to prepare a CA-exendin-4-PEG-Fc conjugate, an immunoglobulin Fc fragment was added to the mono-PEGylated CA-exendin-4 obtained in Example 1-1 to have a total protein concentration of 10 mg/mL to 50 mg/mL, followed by reaction at 4°C to 10°C to room temperature for about 12 to 17 hours. At this time, the reaction solution was 0.1 M potassium phosphate with a pH of 5.5 to 8.5, and 2 mM to 50 mM sodium cyanoborohydride was added as a reducing agent. After the reaction was completed, the CA-exendin-4-PEG-Fc conjugate in the reaction mixture was purified using two kinds of columns, hydrophobic binding and anion exchange columns. The hydrophobic binding column, Source 15Phenyl (Cytiva, USA) isolated and purified the unreacted immunoglobulin Fc fragments using Tris-HCl (pH 7.5) buffer and NaCl concentration gradient, and the anion exchange column, Source 15Q (Cytiva, USA) isolated and purified the CA-exendin-4-PEG-Fc conjugate by removing overreacted impurities using Tris-HCl (pH 7.5) buffer and NaCl concentration gradient.

### Example 2: Preparation of long-acting GLP-2 derivative conjugate

### 2-1. Preparation of CA GLP-2 RK-PEG conjugate

In order to PEGylate the GLP-2 derivative CA GLP-2 RK of SEQ ID NO: 4 in Table 1 with the modified polyethylene glycol ALD (2) PEG (a modified polyethylene glycol in which the terminal hydrogen atoms at both ends are each substituted with a propionaldehyde group (3-oxopropyl group) and the Formula weight of the ethylene glycol repeating unit moiety was 3.4 kDa, manufactured by NOF Corporation, Japan), a reaction was conducted at a molar ratio of the GLP-2 derivative to ALD(2) PEG of 1:5 to 1:20, a concentration of the GLP-2 derivative of 5 mg/mL to 10 mg/mL, and a temperature of 2°C to 8°C for 4 to 16 hours. At this time, the reaction was conducted in 20 mM HEPES buffer with pH 7.5 and ethanol, and 20 mM sodium cyanoborohydride was added as a reducing agent for the reaction. The reaction mixture was subjected to purification of mono-PEGylated GLP-2 derivative using Source 15S (GE, USA) column using a buffer solution comprising sodium citrate with pH 2.0 and ethanol, and potassium chloride concentration gradient.

### 2-2. Preparation of CA GLP-2 RK-PEG-Fc conjugate

Next, the purified mono-PEGylated GLP-2 derivative was reacted with an immunoglobulin Fc fragment at a molar ratio of 1:2 to 1:6. The total protein concentration was adjusted to 30 mg/mL to 35 mg/mL, and the reaction occurred at a temperature of 2°C to 8°C for 12 to 20 hours. During the reaction, 20 mM sodium cyanoborohydride was added as a reducing agent to the reaction solution containing 100 mM potassium phosphate buffer with pH 6.0 and isopropanol.

After completion of the reaction, the reaction mixture was applied to Source 15Q (GE, USA) column using a bis-Tris buffer (pH 6.5 and sodium chloride concentration gradient, and applied to Source 15 ISO (GE, USA) using the concentration gradient of ammonium sulfate and sodium citrate with a pH of 5.0 to 5.2 to purify a long-acting conjugate of the GLP-2 derivative, in which the GLP-2 derivative was covalently linked to the immunoglobulin Fc fragment via a polyethylene glycol linker.

### Example 3: Confirmation of M1 polarization inhibition, macrophage differentiation inhibition, and monocyte migration inhibition in THP-1 cells (monocytes) through combination treatment of long-acting GLP-1 derivative conjugate and long-acting GLP-2 derivative conjugate

An *in vitro* experiment was conducted as follows to confirm the inhibitory effects on M1 polarization, macrophage differentiation, and monocyte migration in THP-1 cells (monocytes) following treatment with the long-acting GLP-1 derivative conjugate, the long-acting GLP-2 derivative conjugate, and the combination treatment of the two conjugates.

### [M1 Polarization Inhibition Confirmation Experiment]

THP-1 cells were treated with long-acting GLP-1 derivative conjugate (0.5 µM or 1 µM), long-acting GLP-2 derivative conjugate (10 µM), and a combination of long-acting GLP-1 derivative conjugate (0.5 µM) and long-acting GLP-2 derivative conjugate (10 µM) for 4 hours. To induce inflammation, lipopolysaccharide (LPS) at 1 µg/mL was treated for 2 hours, and RNA was extracted to measure the mRNA expression levels of the pro-inflammatory cytokines TNFα, IL-1β, and IL-6 (M1 polarization markers) by qPCR.

### [Macrophage Differentiation Inhibition Confirmation Experiment]

THP-1 cells were treated with long-acting GLP-1 derivative conjugate (0.1 µM or 1 µM), long-acting GLP-2 derivative conjugate (10 µM), and a combination of long-acting GLP-1 derivative conjugate (0.1 µM) and long-acting GLP-2 derivative conjugate (10 µM) for 48 hours to induce THP-1 differentiation using phorbol 12-myristate 13-acetate (PMA). The number of differentiated cells, which were adherent cells, was then counted.

### [Monocyte Migration Inhibition Experiment]

THP-1 cells were treated with long-acting GLP-1 derivative conjugate (0.1 µM or 1 µM), long-acting GLP-2 derivative conjugate (10 µM), and a combination of long-acting GLP-1 derivative conjugate (0.1 µM) and long-acting GLP-2 derivative conjugate (10 µM) for 48 hours. The treated THP-1 cells were transferred to the upper chamber of a Boyden chamber, and 50 ng/mL CCL-2 was added to the lower chamber to induce migration, followed by incubation for 4 hours. The extent of migration was measured using a migration assay kit (Abcam).

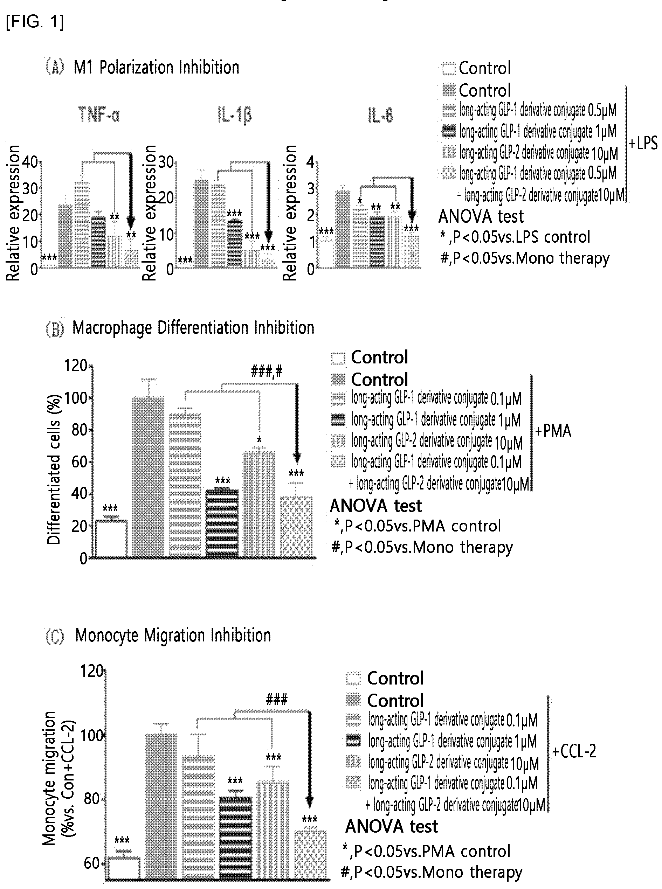

Through the above experiments, the inhibitory effects of each of the long-acting GLP-1 derivative conjugate and the long-acting GLP-2 derivative conjugate on M1 polarization (FIG. 1, (A)), macrophage differentiation (FIG. 1, (B)), and monocyte migration (FIG. 1, (C)) were confirmed, and it was confirmed that these effects were further enhanced when the two were administered in combination.

### Example 4: Examination of small intestine length increase and inflammation reduction in indomethacin-induced inflammatory bowel disease rat models following combined administration of long-acting GLP-1 derivative conjugate, long-acting GLP-2 derivative conjugate, and Anti-TNFα mAb

To measure the *in vivo* efficacy of the increase in small intestine length and inflammation reduction following combined administration of long-acting GLP-1 derivative conjugate, long-acting GLP-2 derivative conjugate, and Anti-TNFα mAb (BioXcell, Catalog #BE0244), an inflammatory bowel disease rat models induced by indomethacin (INN) were used. Specifically, 8-week-old male SD rats (6 rats per group) were divided as follows:
- G1: normal control group (drug-free group, vehicle)
- G2: indomethacin control group (indomethacin 7.5 mg/kg, administered on Days 1 and 2 (D1 and D2))
- G3: group administered with long-acting GLP-2 derivative conjugate (3.1 mg/kg/single) followed by indomethacin administration (long-acting GLP-2 derivative conjugate 3.1 mg/kg/single, D0; indomethacin 7.5 mg/kg, D1 and D2)
- G4: long-acting GLP-1 derivative conjugate administration (0.178 mg/kg/single) followed by indomethacin administration (long-acting GLP-1 derivative conjugate 0.178 mg/kg/single, D0; indomethacin 7.5 mg/kg, D1 and D2)
- G5: group administered with a combination of long-acting GLP-2 derivative conjugate (3.1 mg/kg/single) and long-acting GLP-1 derivative conjugate (0.178 mg/kg/single) followed by indomethacin administration (long-acting GLP-2 derivative conjugate 3.1 mg/kg/single, long-acting GLP-1 derivative conjugate 0.178 mg/kg/single, D0; indomethacin 7.5 mg/kg, D1 and D2)
- G6: group administered with Anti-TNFα mAb (0.2 mg/kg/single) followed by indomethacin administration (Anti-TNFα mAb 0.2 mg/kg/single, D0; indomethacin 7.5 mg/kg, D1 and D2)
- G7: group administered with a combination of long-acting GLP-2 derivative conjugate (3.1 mg/kg/single) and Anti-TNFα mAb (0.2 mg/kg/single) followed by indomethacin administration (long-acting GLP-2 derivative conjugate 3.1 mg/kg/single, Anti-TNFα mAb 0.2 mg/kg/single, D0; indomethacin 7.5 mg/kg, D1 and D2)
- G8: group administered with a combination of long-acting GLP-2 derivative conjugate (3.1 mg/kg/single), long-acting GLP-1 derivative conjugate (0.178 mg/kg/single), and Anti-TNFα mAb (0.2 mg/kg/single) followed by indomethacin administration (long-acting GLP-2 derivative conjugate 3.1 mg/kg/single, long-acting GLP-1 derivative conjugate 0.178 mg/kg/single, Anti-TNFα mAb 0.2 mg/kg/single, D0; indomethacin 7.5 mg/kg, D1 and D2).

In the present specification, the dose of the long-acting GLP-1 derivative conjugate or the long-acting GLP-2 derivative conjugate refers to a value obtained by excluding the mass of the polyethylene glycol linker portion from the total mass of the long-acting GLP-1 derivative conjugate or the long-acting GLP-2 derivative conjugate used, that is, a value based on sum of the masses of the polypeptide portions.

On Day 3, the length of the small intestine and the ulcer area determined by Evans blue staining were measured through necropsy.

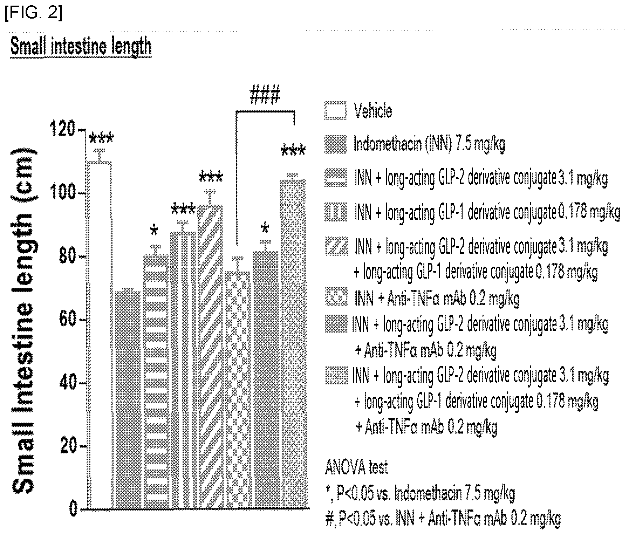

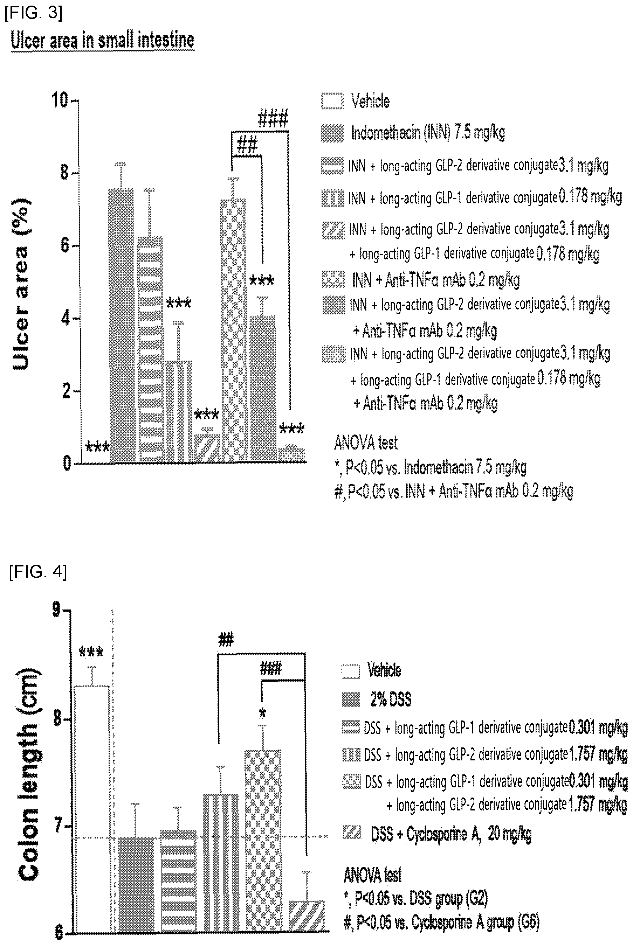

Compared to the indomethacin control group (G2), the group administered with a combination of long-acting GLP-2 derivative conjugate and long-acting GLP-1 derivative conjugate followed by indomethacin administration (G5) showed an increase in small intestine length by 25 cm (FIG. 2) and a reduction in the ulcer area by 6.7% (FIG. 3). Compared to the indomethacin control group (G2), the group administered with a combination of long-acting GLP-2 derivative conjugate and Anti-TNFα mAb followed by indomethacin administration (G7) showed an increase in small intestine length by 12 cm (FIG. 2) and a reduction in the ulcer area by 3.5% (FIG. 3). Additionally, compared to the indomethacin control group (G2), the group administered with a combination of long-acting GLP-2 derivative conjugate, long-acting GLP-1 derivative conjugate, and Anti-TNFα mAb followed by indomethacin administration (G8) showed an increase in small intestine length by 32 cm, which is similar to the normal control group (FIG. 2), and a reduction in the ulcer area by 7.2%, which is also similar to the normal control group (FIG. 3).

### Example 5: Confirmation of colon length increase and reduction in disease activity index (DAI) in dextran sulfate sodium-induced ulcerative colitis mouse model following combined administration of long-acting GLP-1 derivative conjugate and long-acting GLP-2 derivative conjugate

To measure the *in vivo* efficacy of colon length increase and improvement in Disease Activity Index (DAI) following combined administration of long-acting GLP-1 derivative conjugate and long-acting GLP-2 derivative conjugate, a dextran sulfate sodium (DSS)-induced ulcerative colitis mouse model was used. Specifically, 7-week-old male mice (C57BL/6) were divided into groups of 10 mice each as follows. DSS was administered by mixing 2% DSS into the drinking water, where 1 cycle consisted of 5 days of DSS-containing water followed by 5 days of regular water. After a total of 3 cycles, the drug was administered, and the first day of DSS-containing water administration in the first cycle was designated as Day 0.
- G1: Normal control group (drug-free group, vehicle)
- G2: DSS control group (2% DSS, 3 cycles)
- G3: group administered with long-acting GLP-1 derivative conjugate (0.301 mg/kg/Q2D) after DSS administration (2% DSS, 3 cycles; long-acting GLP-1 derivative conjugate 0.301 mg/kg/Q2D, 2 weeks)
- G4: group administered with long-acting GLP-2 derivative conjugate (1.757 mg/kg/Q2D) after DSS administration (2% DSS, 3 cycles; long-acting GLP-2 derivative conjugate 1.757 mg/kg/Q2D, 2 weeks)
- G5: group administered with long-acting GLP-1 derivative conjugate (0.301 mg/kg/Q2D) and long-acting GLP-2 derivative conjugate (1.757 mg/kg/Q2D) after DSS administration (2% DSS, 3 cycles; long-acting GLP-1 derivative conjugate 0.301 mg/kg/Q2D, long-acting GLP-2 derivative conjugate 1.757 mg/kg/Q2D, 2 weeks)
- G6: group administered with cycloclosporine A (20 mg/kg/QD, PO) after DSS administration (2% DSS, 3 cycles; Cyclosporine A 20 mg/kg/QD, 2 weeks)

On Day 44, necropsy was performed, and the length of the colon was measured to assess disease improvement (FIG. 4).

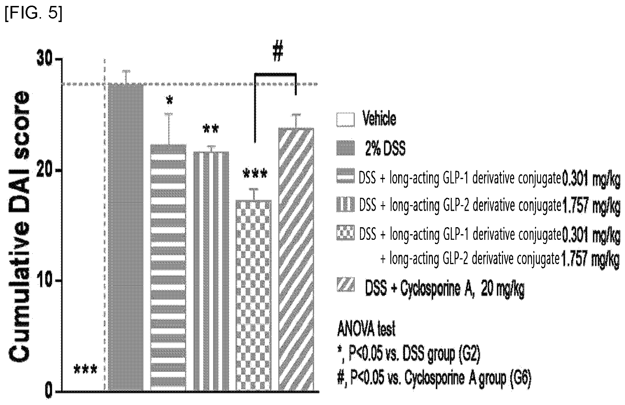

The disease activity index (DAI) for ulcerative colitis was measured by assessing stool consistency and the state of bleeding using scale scores. For stool consistency, the scale scores were given as follows: normal (0 points), stool with visible form but softens upon pressing unlike the normal group (1 point), stool not round and softens easily unlike the normal group (2 points), stool not formed and very soft (3 points), anus wet due to diarrhea with watery discharge instead of solid stool (4 points). For the bleeding state, the scores were as follows: no bleeding (0 points), blood visible upon pressing stool (2 points), blood visible without pressing stool (3 points), and severe bleeding confirmed from the rectum (4 points). Measurements were taken on Days 21, 23, 25, 28, 30, 32, 35, 37, 39, 42, and 44, and the sum of these values was used to assess disease improvement (FIG. 5).

Compared to the DSS control group (G2), the group administered with long-acting GLP-1 derivative conjugate and long-acting GLP-2 derivative conjugate after DSS administration (G5) showed an increase in colon length of approximately 0.8 cm (18%), similar to the normal control group (FIG. 4), and a reduction in the DAI for ulcerative colitis by 10 points (FIG. 5). Additionally, compared to the group administered with Cyclosporine A (20 mg/kg/QD), commonly used in severe clinical stages prior to surgery (G6), the group administered with long-acting GLP-1 derivative conjugate and long-acting GLP-2 derivative conjugate after DSS administration (G5) showed an increase in colon length of approximately 1.4 cm (25%), similar to the normal control group (FIG. 4), and a reduction in the Disease Activity Index by 6 points (FIG. 5).

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the disclosure is defined by the appended claims rather than by the description preceding them, and therefore all changes and modifications that fall within metes and bounds of the claims or equivalents of such metes and bounds are therefore intended to be embraced by the claims.

## Claims

1. A pharmaceutical composition for preventing or treating bowel disease, comprising a pharmaceutically effective amount of GLP-2,
wherein the pharmaceutical composition is administered in combination with an insulinotropic peptide, a TNFα inhibitor, or both.

2. The pharmaceutical composition according to claim 1, wherein the insulinotropic peptide is selected from the group consisting of glucagon-like peptide-1 (GLP-1), exendin-3, exendin-4, an agent, derivative, fragment, and variant thereof, and a combination thereof.

3. The pharmaceutical composition according to claim 1, wherein the insulinotropic peptide is an insulinotropic peptide derivative in which the N-terminal histidine residue of the insulinotropic peptide is substituted with imidazoacetyldeshtidine, desaminohistidine, β-hydroxyimidazopropionyldeshistidine, N-dimethylhistidine, or β-carboxyimidazopropionyldeshistidine.

4. The pharmaceutical composition according to claim 1, wherein the insulinotropic peptide is a native exendin-4; an exendin-4 derivative in which the alpha carbon of the first amino acid of the N-terminus of exendin-4, which is a histidine residue, and the N-terminal amino group attached to the alpha carbon are removed; an exendin-4 derivative in which the N-terminal amino group of exendin-4 is removed; an exendin-4 derivative in which the N-terminal amino group of exendin-4 is substituted with a hydroxy group; an exendin-4 derivative in which the N-terminal amino group of exendin-4 is modified with two methyl groups; an exendin-4 derivative in which the N-terminal amino group of exendin-4 is substituted with a carboxy group; an exendin-4 derivative in which the 12th amino acid (lysine) of exendin-4 is substituted with serine; or an exendin-4 derivative in which the 12th amino (lysine) acid of exendin-4 is substituted with arginine.

5. The pharmaceutical composition according to claim 1, wherein the GLP-2 is a native GLP-2 or a GLP-2 derivative.

6. The pharmaceutical composition according to claim 5, wherein the GLP-2 derivative is a GLP-2 derivative in which a modification selected from the group consisting of substitution, addition, deletion, modification, and a combination thereof has occurred in at least one amino acid of the sequence of the native GLP-2.

7. The pharmaceutical composition according to claim 5, wherein the GLP-2 derivative is a GLP-2 derivative in which a modification has occurred in at least one amino acid of the amino acids at positions 1, 2, 30, and 33 of SEQ ID NO: 1.

8. The pharmaceutical composition according to claim 5, wherein the GLP-2 derivative comprises an amino acid sequence represented by General Formula 1 below:
[General Formula 1] X₁X₂DGSFSDEMNTILDNLAARDFINWLIQTX₃₀ITDX₃₄ (SEQ ID NO: 9)
wherein,
X₁ is histidine, imidazoacetyldeshitidine, desaminohistidine, β-hydroxyimidazopropionyldeshitidine, N-dimethylhistidine, or
β-carboxyimidazopropionyldeshitidine;
X₂ is alanine, glycine, or 2-aminoisobutyric acid (Aib);
X₃₀ is lysine or arginine; and
X₃₄ is absent, or lysine, arginine, glutamine, histidine, 6-azidolysine, or cysteine;
with the proviso that among the amino acid sequences of General Formula 1, a sequence identical to SEQ ID NO: 1 is excluded.

9. The pharmaceutical composition according to claim 8, wherein the GLP-2 derivative comprises an amino acid sequence represented by General Formula 1 in which:
(1) X₁ is imidazoacetyldeshistidine, X₂ is glycine, X₃₀ is lysine, and X₃₄ is cysteine;
(2) X₁ is imidazoacetyldeshistidine, X₂ is glycine, X₃₀ is lysine, and X₃₄ is lysine;
(3) X₁ is imidazoacetyldeshistidine, X₂ is glycine, X₃₀ is arginine, and X₃₄ is lysine;
(4) X₁ is imidazoacetyldeshistidine, X₂ is glycine, X₃₀ is lysine, and X₃₄ is 6-azidolysine;
(5) X₁ is imidazoacetyldeshistidine, X₂ is glycine, X₃₀ is arginine, and X₃₄ is cysteine;
(6) X₁ is imidazoacetyldeshistidine, X₂ is Aib, X₃₀ is lysine, and X₃₄ is cysteine; or
(7) X₁ is histidine, X₂ is Aib, X₃₀ is lysine, and X₃₄ is cysteine.

10. The pharmaceutical composition according to claim 5, wherein the GLP-2 derivative comprises an amino acid sequence represented by General Formula 2 below;
[General Formula 2] X₁X₂DGSFSDEMNTILDNLAARDFINWLIQTX₃₀ITDX₃₄ (SEQ ID NO: 10)
wherein,
X₁ is histidine, imidazoacetyldeshitidine, desaminohistidine, β-hydroxyimidazopropionyldeshitidine, N-dimethylhistidine, or
β-carboxyimidazopropionyldeshitidine;
X₂ is alanine, glycine, or 2-aminoisobutyric acid (Aib);
X₃₀ is lysine or arginine; and
X₃₄ is any one or more amino acids or any one or more amino acids in which a modification has occurred;
with the proviso that among the amino acid sequences of General Formula 2, the sequence identical to SEQ ID NO: 1 is excluded.

11. The pharmaceutical composition according to claim 5, wherein the GLP-2 derivative is a peptide of an amino acid sequence selected from the group consisting of SEQ ID NOS: 2 to 8.

12. The pharmaceutical composition according to claim 1, wherein GLP-2 is administered in combination with an insulinotropic peptide.

13. The pharmaceutical composition according to claim 1, wherein GLP-2 is administered in combination with a TNFα inhibitor.

14. The pharmaceutical composition according to claim 1, wherein GLP-2 is administered in combination with an insulinotropic peptide and a TNFα inhibitor.

15. The pharmaceutical composition according to claim 1, wherein the TNFα inhibitor is a soluble TNF receptor, an anti-TNFα inhibitor or a fragment thereof, or a combination thereof.

16. The pharmaceutical composition according to claim 1, wherein the bowel disease is at least one selected from the group consisting of irritable bowel disease, enteritis, inflammatory bowel disease, enterocolitis, colitis, pancreatitis, ileitis, bowel atrophy, and bowel damage.

17. The pharmaceutical composition according to claim 16, wherein the inflammatory bowel disease is at least one selected from the group consisting of ulcerative colitis, Crohn's disease, and Behcet's disease.

18. The pharmaceutical composition according to claim 1, wherein the composition, when administered to a subject, causes at least one among inhibition of M1 polarization in monocytes, inhibition of macrophage differentiation, and inhibition of monocyte migration.

19. The pharmaceutical composition according to claim 1, wherein the composition, when administered to a subject, causes at least one among increased length of the small intestine, decreased inflammation of the small intestine, increased length of the large intestine, and decreased inflammation of the large intestine.

20. The pharmaceutical composition according to claim 1, wherein in the insulinotropic peptide and GLP-2, the C-terminus is not modified or amidated.

21. The pharmaceutical composition according to claim 1, wherein:
(i) the insulinotropic peptide is in the form of a long-acting conjugate to which a biocompatible material capable of increasing its *in vivo* half-life is bound;
(ii) the GLP-2 is in the form of a long-acting conjugate to which a biocompatible material capable of increasing its *in vivo* half-life is bound; or
(iii) the insulinotropic peptide and GLP-2 are each in the form of a long-acting conjugate to which a biocompatible material capable of increasing their respective in *vivo* half-life is bound.

22. The pharmaceutical composition according to claim 21, wherein the conjugate is represented by Formula 1 below:
[Formula 1] X - La - F
wherein,
X is an insulinotropic peptide or GLP-2;
L is a linker containing an ethylene glycol repeating unit;
a is 0 or a natural number wherein when a is 2 or more, each L is independent of the other(s);
F is an immunoglobulin Fc region; and
the "-" represents a covalent bond.

23. The pharmaceutical composition according to claim 22, wherein the immunoglobulin Fc region is an aglycosylated IgG4 Fc region.

24. The pharmaceutical composition according to claim 22, wherein the F is a dimer consisting of two polypeptide chains, and one end of L is linked to only one of the two polypeptide chains.

25. The pharmaceutical composition according to claim 22, wherein the L is polyethylene glycol.

26. The pharmaceutical composition according to claim 22, wherein the formula weight of the ethylene glycol repeating unit moiety in L is in the range of 1 kDa to 100 kDa.

27. The pharmaceutical composition according to claim 1, wherein the composition further comprises a pharmaceutically acceptable carrier, an excipient, or a diluent.

28. The pharmaceutical composition according to any one of claims 1 to 27, wherein (i) GLP-2 and an insulinotropic peptide; (ii) GLP-2 and a TNFα inhibitor; or (iii) GLP-2, an insulinotropic peptide, and a TNFα inhibitor are administered in combination simultaneously, sequentially, or in reverse order.
